# EUROPEAN PATENT APPLICATION

(11) **EP 4 238 572 A1**
(43) Date of publication of application: **06.09.2023**
(21) Application number: 21886286.0
(22) Date of filing: 27.10.2021
(51) Int. Cl.: A61K 35/19, A61K 38/17, A61L 27/38, A61P 19/00, C12N 5/0775, C12N 5/078, C12N 5/16, C12N 15/12, C07K 14/47

(54) **OSTEOGENIC COMPOSITION AND USE THEREOF**

(30) Priority: 27.10.2020 JP 2020180042
(71) Applicant: NAGASAKI UNIVERSITY, Nagasaki 852-8521 (JP); Megakaryon Corporation, Kyoto-shi, Kyoto 600-8813 (JP)
(72) Inventor: IWATAKE Mayumi, Nagasaki-shi, Nagasaki 852-8521 (JP); SUMITA Yoshinori, Nagasaki-shi, Nagasaki 852-8521 (JP); ASAHINA Izumi, Nagasaki-shi, Nagasaki 852-8521 (JP); SATAKE Makoto, Kyoto-shi, Kyoto 600-8813 (JP); HAZAMA Yasuko, Kyoto-shi, Kyoto 600-8813 (JP)
(74) Representative: Schön, Christoph
(86) International application number: PCT/JP2021/039716
(87) International publication number: WO 2022/092169

(57) **Abstract**

A composition having cell-derived physiological activity is provided. An osteogenic composition of the present invention includes a treated product obtained by treating megakaryocytes or treating a culture thereof.

## Description

### TECHNICAL FIELD

The present invention relates to an osteogenic composition and use thereof.

### BACKGROUND ART

Attempts have been made to develop cell preparations using cells that facilitate tissue regeneration, such as mesenchymal stem cells, as therapeutic agents for regenerative medicine.

It is assumed that the cells that facilitate tissue regeneration release proteins having physiological activity, such as growth factors, and thereby promote tissue regeneration.

In regenerative medicine within the field of dentistry, platelet-rich plasma is used in bone augmentation (bone formation) treatment, etc. However, because platelet-rich plasma is prepared by separation and centrifugal concentration from isolated human blood, the number of platelets in platelet-rich plasma is not standardized, and thus, the efficacy thereof is unclear.

### SUMMARY OF INVENTION

### Technical Problem

Therefore, it is an object of the present invention to provide a composition having cell-derived osteogenic activity.

### Solution to Problem

To achieve the aforementioned object, the osteogenic composition (hereinafter, also referred to as a "composition") of the present invention includes a treated product obtained by treating megakaryocytes or treating a culture thereof.

An osteogenic kit (hereinafter, also referred to as a "kit" ) of the present invention includes an osteogenic composition and a scaffold material for osteogenesis, and the osteogenic composition is the osteogenic composition of the present invention.

### Advantageous Effects of Invention

With the present invention, it is possible to provide a composition having cell-derived osteogenic activity.

### Brief Description of Drawings

[FIG. 1] FIG. 1 is a schematic diagram showing the configuration of a concentration system in Example 1.
[FIG. 2] FIG. 2 shows graphs illustrating cell-proliferative activity in Example 1, with FIG. 2(A) illustrating relative values of the proliferative activity, and FIG. 2(B) illustrating the doubling time.
[FIG. 3] FIG. 3 shows photographs of images obtained through alkaline phosphatase staining in Example 1.
[FIG. 4] FIG. 4 shows photographs of images obtained through calcium staining in Example 1.
[FIG. 5] FIG. 5 is a graph illustrating the concentrations of calcium ions in the culture media in Example 1.
[FIG. 6] FIG. 6 shows graphs illustrating cell-proliferative activity in Example 2, with FIG. 6(A) illustrating relative values of the proliferative activity, and FIG. 6(B) illustrating the doubling time.
[FIG. 7] FIG. 7 shows photographs of images obtained through calcium staining in Example 2.
[FIG. 8] FIG. 8 shows photographs of images obtained through calcium staining in Example 2.
[FIG. 9] FIG. 9 is a graph illustrating the concentrations of calcium ions in the culture media in Example 2.
[FIG. 10] FIG. 10 is a graph illustrating the number of cells in Example 3.
[FIG. 11] FIG. 11 shows graphs illustrating ALP activity in Example 3.
[FIG. 12] FIG. 12 is a graph illustrating the number of cells in Example 3.
[FIG. 13] FIG. 13 shows graphs illustrating ALP activity in Example 3.
[FIG. 14] FIG. 14 shows photographs illustrating osteogenesis in Example 4.
[FIG. 15] FIG. 15 shows graphs illustrating the osteogenesis level or bone augmentation level in Example 4.
[FIG. 16] FIG. 16 shows a schematic diagram and graphs illustrating the overview of an experiment and cytokine gene-expression levels in Example 5.
[FIG. 17] FIG. 17 shows a schematic diagram and graphs illustrating the overview of an experiment and cytokine gene-expression levels in Example 5.
[FIG. 18] FIG. 18 shows photographs illustrating osteogenesis in the fourth week after transplantation in Example 6.
[FIG. 19] FIG. 19 shows graphs illustrating bone volumes, bone mineral contents, ratios between a bone defect volume and a new bone volume, and bone mineral densities in the fourth week after transplantation in Example 6.

### DESCRIPTION OF EMBODIMENTS

### Osteogenic Composition

As described above, an osteogenic composition of the present invention includes a treated product obtained by treating megakaryocytes or treating a culture thereof. The osteogenic composition of the present invention is characterized by including a treated product obtained by treating megakaryocytes or treating a culture thereof, and there is no particular limitation on configurations and conditions other than this. The composition of the present invention can be used to, for example, provide a composition having cell-derived physiological activity. The composition of the present invention is capable of promoting, for example, differentiation to osteoblasts and thus inducing osteogenesis.

In the present invention, "osteogenesis" may be used to mean an increase in the volume of already-existing bone (i.e., "bone augmentation" (bone formation)), formation of new bone (i.e., "new bone formation"), or recovery of lost (defect) bone or a portion thereof (i.e., "bone regeneration"). Accordingly, the osteogenic composition of the present invention can also be referred to as a "bone augmentation (bone formation) composition", "new bone formation composition", and/or "bone regeneration composition".

It is assumed that the composition of the present invention promotes, accelerates (i.e., promotes induction), accentuates, intensifies, and/or strengthens the induction of osteoblast differentiation and thus, induces osteogenesis, as described later. Accordingly, the composition of the present invention can also be referred to as a composition for promoting, accelerating (i.e., promoting induction), accentuating, intensifying, and/or strengthening the induction of osteoblast differentiation, or a composition for inducing, promoting, accelerating (i.e., promoting induction), accentuating, intensifying, and/or strengthening osteogenesis.

In the present invention, the term "megakaryocyte" means a cell that is the largest bone marrow cell in a living organism and releases platelets, as well as a cell having an equivalent function. The term "cell having an equivalent function" means a cell having an ability to produce platelets. In the present invention, megakaryocytes may be megakaryocytes before multinucleation (polyploid formation), namely immature megakaryocytes or megakaryocytes in the proliferative phase, or megakaryocytes after multinucleation (multinucleated megakaryocytes). Specifically, the megakaryocytes may be, for example, promegakaryoblasts, megakaryoblasts, promegakaryocytes, or mature megakaryocytes. It is sufficient that the number of chromosome sets in the megakaryocytes after multinucleation exceeds 2, and specifically 16 to 32.

Although there is no particular limitation on the source of the megakaryocytes, examples thereof include a human and a non-human animal. Examples of non-human animals include primates such as a monkey, a gorilla, a chimpanzee, a marmoset, a mouse, a rat, a dog, a cat, a rabbit, a sheep, a horse, and a guinea pig. The source of the megakaryocytes may be of, for example, the same type as or a different type from a subject to which the composition of the present invention is administered.

In the present invention, the megakaryocytes can be identified based on a cell surface marker. In the case where the megakaryocytes are derived from a human, examples of the cell surface marker include CD41a, CD42a, and CD42b. That is to say, the megakaryocytes are cells positive for CD41a, CD42a, and CD42b. In the case where the megakaryocytes are derived from a human, the cell surface marker may be, for example, at least one selected from the group consisting of CD9, CD61, CD62p, CD42c, CD42d, CD49f, CD51, CD110, CD123, CD131, and CD203c.

The megakaryocytes may be megakaryocytes isolated from a living organism or megakaryocytes induced from cells that are less differentiated than megakaryocytes (hereinafter, also referred to as "progenitor cells"), such as pluripotent cells. The term "cells that are less differentiated than megakaryocytes" means cells capable of differentiating to the megakaryocytes.

In the case of isolating megakaryocytes from a living organism, the megakaryocytes can be isolated from, for example, the bone marrow since megakaryocytes are present in the bone marrow. In this case, the megakaryocytes may include other cells derived from the living organism.

In the case of inducing megakaryocytes from progenitor cells, the megakaryocytes can be induced *in vitro* as described later. In this case, the megakaryocytes may include the progenitor cells. Examples of the progenitor cells include hematopoietic stem cells, hematopoietic progenitor cells, CD34-positive cells, megakaryocyte-erythroid progenitor (MEP) cells, and megakaryocyte progenitor cells. For example, the progenitor cells may be isolated from the bone marrow, cord blood, peripheral blood, and the like, or induced from pluripotent cells such as ES cells (embryonic stem cells), induced pluripotent stem cells (iPS cells), nuclear transplantation ES cells (ntES cells), germinal stem cells, somatic stem cells, and embryonic tumor cells.

In the case of inducing megakaryocytes from progenitor cells, the megakaryocytes are preferably immortalized megakaryocytes. For example, the homogeneity of the immortalized megakaryocytes in the cell differentiation phase is higher than that of megakaryocytes induced using other megakaryocyte induction methods, thus making it possible to suppress a variation between the component compositions of obtained treated products. For example, the immortalized megakaryocytes are induced by introducing an oncogene and a polycomb gene, or an oncogene, a polycomb gene, and an apoptosis inhibitor gene, into the progenitor cells, as described later.

The term "oncogene" means a gene capable of inducing the canceration of cells in a living organism, and examples of the oncogene include the MYC family genes such as c-MYC, N-MYC, L-MYC, the SRC family genes, the RAS family genes, the RAF family genes, and the protein kinase family genes such as c-kit (CD 117), PDGFR (platelet-derived growth factor receptor), and Abl (Abelson murine leukemia viral oncogene homolog).

The term "polycomb gene" means a gene known to have a function of avoiding cellular senescence by negatively regulating CDKN2a (cyclin-dependent kinase inhibitor 2A, INK4a/ARF) (References 1 to 3 below). Specific examples of the polycomb gene include BMI1 (Polycomb complex protein BMI-1, polycomb group RING finger protein 4 (PCGF4), RING finger protein 51 (RNF51)), Mel18 (Polycomb group RING finger protein 2), Ring (Ring Finger Protein) 1a/b, Phc (Polyhomeotic Homolog) 1/2/3, Cbx (Chromobox) 2/4/6/7/8, Ezh2 (Enhancer Of Zeste 2 Polycomb Repressive Complex 2 Subunit), Eed (Embryonic Ectoderm Development), Suz12 (SUZ12 Polycomb Repressive Complex 2 Subunit), HADC (Histone deacetylases), and Dnmt (DNA (cytosine-5)-methyltransferase)1/3a/3b.
Reference 1: Hideyuki Oguro et.al., "Senescence and aging of stem cells regulated by polycomb complexes", Regenerative Medicine, 2007, vol.6, No.4, pages 26-32
Reference 2: Jesus Gil et.al, "Regulation of the INK4b-ARF-INK4a tumour suppressor locus: all for one or one for all", Nature Reviews Molecular Cell Biology, 2007, vol.7, pages 667-677
Reference 3: Soo-Hyun Kim et.al., "Absence of p16INK4a and truncation of ARF tumor suppressors in chickens", PNAS, 2003, vol. 100, No. 1, pages 211-216

The term "apoptosis inhibitor gene" means a gene capable of functioning to inhibit cellular apoptosis, and examples thereof include BCL2 (B-cell lymphoma 2), Bcl-xL (B-cell lymphoma-extra large), Survivin (Baculoviral IAP Repeat Containing 5), and MCL1 (BCL2 Family Apoptosis Regulator).

The immortalized megakaryocytes are preferably megakaryocytes including the BMI1 gene, the MYC gene, and the Bcl-xL gene, which are exogenous genes. The term "exogenous" means that the gene is introduced into a cell from the outside of the cell. The exogenous gene may be located on the chromosome of the cell, or may be located in the nucleus or the cytoplasm. The exogenous gene can be detected by, for example, measuring the gene number. In the case where the gene is an autosomal gene, the gene number per cell is two since the gene number per autosome is one. Accordingly, in the case where the exogenous gene is not present, the detected gene number per cell is two. On the other hand, in the case where the exogenous gene is present, the detected gene number per cell is three or more. In this case, the exogenous gene can be detected using, for example, PCR with primers, probes, or a combination thereof. In the case where the exogenous gene has a tag sequence or selective marker, the exogenous gene may be detected by detecting the tag sequence or selective marker. Also, the exogenous gene can be detected, for example, using an antibody or the like against a protein translated from the gene.

The culture of megakaryocytes is produced by, for example, culturing the above-mentioned megakaryocytes. The culturing of megakaryocytes can be carried out by, for example, culturing the megakaryocytes in the presence of a culture medium as described later.

The culture of megakaryocytes is obtained by culturing the megakaryocytes, and is thus a mixture that includes, for example, the megakaryocytes and platelets produced by the megakaryocytes as cellular components. The term "cellular components" means cells and platelets. As described above, the megakaryocytes can be induced from the cells that are less differentiated than megakaryocytes. Accordingly, when megakaryocytes induced from the cells that are less differentiated than megakaryocytes are included as megakaryocytes for producing the culture of megakaryocytes, the culture of megakaryocytes may include the cells that are less differentiated than megakaryocytes.

In the present invention, the culture of megakaryocytes may be a culture itself obtained by culturing the megakaryocytes, or a product obtained by manipulating the culture. Examples of the culture manipulation include removal of a liquid fraction, extraction of a cellular component fraction, and a change in the composition of the cellular components that include platelets. Examples of the change in the composition of the cellular components include removal of cells and/or platelets from the mixture, extraction of cells and/or platelets from the mixture, and addition of cells and/or platelets to the mixture.

The term "platelet" means a cellular component that is one of the cellular components in blood and is positive for CD41a and CD42b. In an example, the platelets do not have a cell nucleus and are smaller than the megakaryocytes. Accordingly, the platelets and the megakaryocytes can be distinguished based on, for example, the presence or absence of cell nuclei, and/or the size. It is known that the platelets play an important role in thrombogenesis and hemostasis, and contribute to the regeneration of a damaged tissue and the pathologic physiology of inflammation. It is also known that, when platelets are activated in response to bleeding or the like, receptors of cell adhesion factors such as Integrin αIIBβ3 (glycoprotein IIb/IIIa; complex of CD41a and CD61) are expressed on the membranes of the platelets. Also, when the platelets are activated, the platelets clump together, and various blood coagulation factors released from the platelets cause fibrin coagulation. Thus, blood clots are formed, and hemostasis progresses. In the present invention, the source of the platelets is the same as the source of the megakaryocytes.

In the present invention, the treated product may be prepared from the megakaryocytes or the culture of megakaryocytes. In the case where the treated product is prepared from the culture of megakaryocytes, the culture of megakaryocytes may be manipulated. Specifically, the treated product may be, for example, a treated product obtained by treating the megakaryocytes or treating a cellular fraction of a culture thereof, or a treated product obtained by treating a manipulated product formed by manipulating the megakaryocytes or manipulating a culture thereof. There is no particular limitation on the treatment for preparing the treated product, and examples of the treatment include: treatment for changing the density of the cellular component, such as concentrating; extraction treatment for extracting the cellular component, such as drying, freezing, freeze-drying, solvent treatment, surfactant treatment, enzyme treatment, and protein-fraction extraction treatment; and disruption treatment such as grinding and pulverizing. Specific examples of the treated product include: an extract of the megakaryocytes or a culture thereof, such as a concentrate, a dried product, a frozen product, a freeze-dried product, a solvent-treated product, a surfactant-treated product, an enzyme-treated product, a fractionated protein, and an ultrasonic wave-treated product; a product obtained by performing the disruption treatment on the megakaryocytes or a culture thereof, such as a ground product and a pulverized product; an extract of a cellular fraction of the megakaryocytes or a culture thereof, such as a concentrate, a dried product, a frozen product, a freeze-dried product, a solvent-treated product, a surfactant-treated product, an enzyme-treated product, a fractionated protein, and an ultrasonic wave-treated product; and a product obtained by performing the disruption treatment on a cellular fraction of the megakaryocytes or a culture thereof, such as a ground product and a pulverized product. The treated product may be constituted by one type of treated product, or may be a mixture of two or more types of treated products. There is no particular limitation on the mixture, and a mixture obtained by mixing a combination of any treated products at any ratio can be used.

The treated product includes, for example, one or more growth factors and/or one or more growth factor receptors. Also, the treated product has, for example, physiological activity such as cell proliferation-promoting activity or osteoblast differentiation-promoting activity. Furthermore, as described above, the treated product can be manufactured by, for example, treating the megakaryocytes or a culture thereof. Accordingly, in the present invention, the treated product can be defined by, for example, Conditions (1) to (3) below. For example, the treated product may be defined by any of Conditions (1) to (3) below, two or more of the conditions, or all of the conditions. Specifically, the treated product can be defined by, for example, a combination of the conditions.

### Conditions

(1) Contents of growth factor and/or growth factor receptor;
(2) Physiological activity of treated product;
(3) Method for manufacturing treated product

### Combinations of Conditions

Condition (1), (2), or (3);
Conditions (1) and (2), Conditions (1) and (3), or Conditions (2) and (3);
Conditions (1), (2), and (3)

### (1) Condition (1)

As described above, Condition (1) relates to the content of a growth factor and/or a growth factor receptor. Condition (1) above may define the content of a growth factor or the content of a growth factor receptor, or the content of a growth factor and the content of a growth factor receptor. One type or two or more types of growth factors may be used in the definition by Condition (1) above. One type or two or more types of growth factor receptors may be used in the definition by Condition (1) above.

In Condition (1) above, examples of the growth factor include a basic fibroblast growth factor (bFGF), an insulin-like growth factor-binding protein-1 (IGFBP-1), an insulin-like growth factor-binding protein-2 (IGFBP-2), an insulin-like growth factor-binding protein-3 (IGFBP-3), an insulin-like growth factor-binding protein-6 (IGFBP-6), a placental growth factor (PIGF), a vascular endothelial growth factor (VEGF), an endocrine-gland-derived vascular endothelial growth factor (EG-VEGF), a growth differentiation factor-15 (GDF-15), amphiregulin (AR), a bone morphogenetic protein-5 (BMP-5), a bone morphogenetic protein-7 (BMP-7), a hepatic growth factor (HGF), and TGF β1 (transforming growth factor β1).

In Condition (1) above, examples of the growth factor receptor include a stem cell factor receptor (SCFR), an epidermal growth factor receptor (EGFR), and a vascular endothelial growth factor receptor 2 (VEGFR2).

The contents may be, for example, the weights of the growth factor and the growth factor receptor in the treated product, or the weights of the growth factor and the growth factor receptor with respect to the total protein weight in the treated product (contents per total protein), and the latter is preferable.

The total protein weight can be determined using, for example, the BCA protein assay. The BCA protein assay is a protein assay in which a coordination bond between a monovalent copper ion and two molecules of bicinchoninic acid is utilized. It is preferable that samples to be subjected to the BCA protein assay do not include, for example, a reducing agent and/or a copper ion-chelating agent. The BCA protein assay can be carried out in conformity with Reference 4 below, and, for example, a commercially available kit or the like may be used. Pierce^{™} BCA Protein Assay Kit (manufactured by Thermo Fisher Scientific) and the like can be used as a kit of the BCA protein assay.

Reference 4: Toshiharu Hase et.al., "Basic Principles and Experiments in Protein Science 1, Protein Production: Extraction, Purification, and Synthesis" Kagaku-Dojin Publishing Company, Inc., December 13, 2008

The total protein concentration in the treated product can be set as appropriate by, for example, adjusting the number of cells to be subjected to treatment and the volume of a solvent in the treated product. The total protein concentration in the treated product can be relatively increased by, for example, increasing the number of cells to be subjected to treatment, reducing the volume of a solvent in the treated product, or extracting proteins from the megakaryocytes. Also, the total protein concentration in the treated product can be relatively reduced by, for example, reducing the number of cells to be subjected to treatment, increasing the volume of a solvent in the treated product, or extracting proteins from the culture of megakaryocytes. Specifically, in the case where the number of cells to be subjected to the treatment is 1× 10⁸ and the volume of a solvent in the treated product is 100 µl, the total protein concentration in the treated product is, for example, 0.1 to 200 mg/ml. The solvent is, for example, an aqueous solvent, which will be described later.

The weights of the growth factor and the growth factor receptor can be determined using, for example, sandwich ELISA. The sandwich ELISA can be carried out in conformity with Reference 5 below, and, for example, a commercially available kit or the like may be used. Quantibody^{®} Human Growth Factor Array 1 (manufactured by RayBiotech) and the like can be used as a kit of the sandwich ELISA.

Reference 5: Biochemical Assay Society of JAPAN, ed. "Immunoassay: from Basics to Advanced Techniques", Kodansha Ltd., December 20, 2014

The content of the growth factor and the content of the growth factor receptor in the treated product, for example, are as follows.

In the case where the growth factor is bFGF, the treated product includes bFGF in an amount of, for example, 2000 to 20000 pg, 5000 to 20000 pg, or 10000 to 20000 pg per 1 mg of the total protein. The treated product exhibits cell proliferation-promoting activity and activity to promote differentiation to osteoblast as described later, for example, due to bFGF being contained.

In the case where the growth factor is IGFBP-1, the treated product includes IGFBP-1 in an amount of, for example, 0 to 200 pg, 0.01 to 200 pg, 0.01 to 100 pg, or 0.01 to 50 pg per 1 mg of the total protein.

In the case where the growth factor is IGFBP-2, the treated product includes IGFBP-2 in an amount of, for example, 8000 to 80000 pg, 10000 to 80000 pg, or 20000 to 80000 pg per 1 mg of the total protein.

In the case where the growth factor is PIGF, the treated product includes PIGF in an amount of, for example, 1 to 60 pg, 1 to 30 pg, or 1 to 20 pg per 1 mg of the total protein.

In the case where the growth factor is VEGF, the treated product includes VEGF in an amount of, for example, 20 to 800 pg, 20 to 600 pg, or 20 to 400 pg per 1 mg of the total protein.

In the case where the growth factor is GDF-15, the treated product includes GDF-15 in an amount of, for example, 1000 to 10000 pg, 1000 to 5000 pg, or 2000 to 5000 pg per 1 mg of the total protein.

In the case where the growth factor is AR, the treated product includes AR in an amount of, for example, 0 to 16 pg, 0.01 to 16 pg, 0.1 to 16 pg, or 1 to 16 pg per 1 mg of the total protein.

In the case where the growth factor is HGF, the treated product includes HGF in an amount of, for example, 0 to 100 pg, 0.01 to 100 pg, 0.01 to 50 pg, or 0.01 to 30 pg per 1 mg of the total protein.

In the case where the growth factor is BMP-7, the treated product includes BMP-7 in an amount of, for example, 0 to 1000 pg or 0.01 to 1000 pg per 1 mg of the total protein.

In the case where the growth factor receptor is SCFR, the treated product includes SCFR in an amount of, for example, 200 to 2000 pg, 300 to 1500 pg, or 400 to 1000 pg per 1 mg of the total protein.

In the case where the growth factor receptor is EGFR, the treated product includes EGFR in an amount of, for example, 0 to 60 pg, 0.01 to 60 pg, 1 to 50 pg, 1 to 45 pg, or 10 to 40 pg per 1 mg of the total protein.

In the case where the growth factor receptor is VEGFR2, the treated product includes VEGFR2 in an amount of, for example, 20 to 400 pg, 50 to 350 pg, or 100 to 300 pg per 1 mg of the total protein.

As described above, the contents of one type or two or more types of growth factors are used in the definition by Condition (1) above, or the contents of one type or two or more types of growth factor receptors are used in the definition, or these contents may be used in any combination in the definition. In this case, for example, at least 1, 2, 3, 4, 5, 6, 7, 8, 9, 10, 11, or 12 conditions selected from the group consisting of Conditions (A1) to (A9) and (B1) to (B3) below are used in the definition by Conditions (1) above. Specific examples of combinations of the content of the growth factor and/or the content of the growth factor receptor include the following combinations.

### Combinations of Content of Growth Factor and/or Content of Growth Factor Receptor

Any one of conditions (A1) to (A9) and (B1) to (B3):
(A1) content of bFGF, (A2) content of IGFBP-1, (A3) content of IGFBP-2, (A4) content of PIGF, (A5) content of VEGF, (A6) content of GDF-15, (A7) content of AR, (A8) content of HGF, (A9) content of BMP-7, (B1) content of SCFR, (B2) content of EGFR, or (B3) content of VEGFR2;

Any two of conditions (A1) to (A9) and (B1) to (B3):
(A1) and (A2), (A1) and (A3), (A1) and (A4), (A1) and (A5), (A1) and (A6), (A1) and (A7), (A1) and (A8), (A1) and (A9), (A1) and (B1), (A1) and (B2), (A1) and (B3),
(A2) and (A3), (A2) and (A4), (A2) and (A5), (A2) and (A6), (A2) and (A7), (A2) and (A8), (A2) and (A9), (A2) and (B1), (A2) and (B2), (A2) and (B3),
(A3) and (A4), (A3) and (A5), (A3) and (A6), (A3) and (A7), (A3) and (A8), (A3) and (A9), (A3) and (B1), (A3) and (B2), (A3) and (B3),
(A4) and (A5), (A4) and (A6), (A4) and (A7), (A4) and (A8), (A4) and (A9), (A4) and (B1), (A4) and (B2), (A4) and (B3),
(A5) and (A6), (A5) and (A7), (A5) and (A8), (A5) and (A9), (A5) and (B1), (A5) and (B2), (A5) and (B3),
(A6) and (A7), (A6) and (A8), (A6) and (A9), (A6) and (B1), (A6) and (B2), (A6) and (B3),
(A7) and (A8), (A7) and (A9), (A7) and (B1), (A7) and (B2), (A7) and (B3),
(A8) and (A9), (A8) and (B1), (A8) and (B2), (A8) and (B3),
(A9) and (B1), (A9) and (B2), (A9) and (B3),
(B1) and (B2), (B1) and (B3), or
(B2) and (B3).

The contents per total protein may also be adjusted by, for example, adding or removing another protein different from the growth factor and the growth factor receptor in accordance with the application of the composition of the present invention. Examples of the other protein include proteins that have no influence on the activity of the growth factor and the growth factor receptor, and specific examples thereof include serum albumin such as human serum albumin and gamma globulin such as human gamma globulin. Examples of the removal of a protein include removal using a column, and removal using an antibody.

### (2) Condition (2)

As described above, Condition (2) relates to the physiological activity of the treated product. In Condition (2) above, the term "physiological activity of the treated product" means activity to regulate a function of a cell, a tissue, or an organ. Examples of the function of a cell include proliferation and differentiation.

Examples of the physiological activity of the treated product include cell proliferation-promoting activity, activity to promote differentiation to osteoblasts, and activity to promote differentiation of osteo-progenitor cells to osteoblasts. There is no particular limitation on the target cells of the cell proliferation-promoting activity, and an example of the cells is mesenchymal stem cells. Examples of the osteo-progenitor cells targeted by the activity to promote differentiation to osteoblasts include mesenchymal stem cells and osteoblasts. The treated product may have, for example, one type of activity or a plurality of types of activity.

The mesenchymal stem cells have a self-replicating ability and are capable of differentiating to bones, cartilages, and fat cells. The mesenchymal stem cells can be identified based on a cell surface marker. The mesenchymal stem cells are, for example, positive for CD73, CD90, and CD105 and negative for CD14, CD34, and CD45.

The osteoblasts are capable of synthesizing and secreting bone matrices. The osteoblasts can be identified as, for example, cells positive for alkaline phosphatase and osteocalcin.

Regarding the cell proliferation-promoting activity, it is sufficient that the cell-proliferative ability improves, for example, compared with a control group that is prepared in the same manner except that the composition of the present invention is not added thereto, and the cell-proliferative ability may decrease, for example, compared with the initial level. In this case, the "proliferation-promoting activity" can also be referred to as, for example, "inhibition of a decrease in proliferative activity". Specifically, the proliferative activity of the mesenchymal stem cells decreases as the cells are repeatedly subjected to subculture. The composition of the present invention is capable of inhibiting a decrease in the mesenchymal stem cell-proliferative activity, and therefore, it can be said that the composition of the present invention exhibits proliferation-promoting activity. The cell proliferation-promoting activity can be measured, for example, under culture conditions where the target cells proliferate. The culture conditions can be set as appropriate, for example, in accordance with the cell type.

Regarding the activity to promote differentiation to osteoblasts, it is sufficient that an ability to differentiate to osteoblasts improves, for example, compared with a control group that is prepared in the same manner except that the composition of the present invention is not added thereto. The term "ability to differentiate improves" may mean, for example, that the rate of differentiation to osteoblasts increases, or that the ratio of cells that differentiate to osteoblasts increases. Regarding the differentiation to osteoblasts, JP 2012-120529A, for example, can be referred to, for example. Specifically, a method for inducing osteoblasts from mesenchymal stem cells can be carried out based on Example 2, which will be described later.

### (3) Condition (3)

As described above, Condition (3) relates to a method for manufacturing a treated product. The description of a method for manufacturing the composition of the present invention, which will be described later, can be applied to the method for manufacturing a treated product to be used in the composition of the present invention.

The composition of the present invention may be used in vitro or *in vivo.*

In the case where the composition of the present invention is used *in vitro,* examples of the administration subject include cells, tissues, and organs, and examples of the cells include cells collected from a living organism, and cultured cells.

In the case where the composition of the present invention is used *in vivo,* examples of the administration subject include a human and a non-human animal other than a human. Examples of the non-human animal include a mouse, a rat, a rabbit, a dog, a sheep, a horse, a cat, a goat, a monkey, and a guinea pig.

There is no particular limitation on the use conditions (administration conditions) of the composition of the present invention, and the administration form, the timing of administration, the dosage amount, and the like can be determined as appropriate, for example, in accordance with the type of administration subject, etc.

In the case where the composition of the present invention is used *in vitro,* the composition of the present invention can be added to, for example, a culture medium of target progenitor cells. The composition of the present invention may be added to a maintenance culture medium used to maintain the progenitor cells, or to a differentiation culture medium used to differentiate the progenitor cells to the osteoblasts, or to both the maintenance culture medium and the differentiation culture medium. With the composition of the present invention, for example, by maintaining the progenitor cells in a maintenance culture medium containing the composition, it is possible to also promote differentiation when the progenitor cells subsequently differentiate to osteoblasts. The final concentration of the total protein derived from the composition of the present invention in the maintenance culture medium is, for example, 10 to 1000 µg/ml, 10 to 500 µg/ml, or 10 to 300 µg/ml. The final concentration of the total protein derived from the composition of the present invention in the differentiation culture medium is, for example, 10 to 1000 µg/ml, 10 to 500 µg/ml, or 10 to 300 µg/ml.

In the case where the composition of the present invention is used *in vivo,* the dosage amount of the composition can be determined as appropriate, for example, in accordance with the type, condition, and age of the administration subject, the administration method, and the like. Specifically, in the case of administration to a mouse, although the sum of the amounts of all the proteins derived from the composition is not particularly limited and the dosage amount of the composition per day can be determined as appropriate, for example, in accordance with the application thereof, specific example of the dosage amount of the composition per day is 10 ng to 100 mg. In the case of administration to a human, although the sum of the amounts of all the proteins derived from the composition per day is not particularly limited and the dosage amount of the composition can be determined as appropriate, for example, in accordance with the application thereof, specific example of the dosage amount of the composition per day is 10 ng to 100 g. The administration frequency per day is, for example, 1 to 5 times, 1 to 3 times, or once or twice.

There is no particular limitation on the administration form of the composition of the present invention. In the case where the composition of the present invention is administered *in vivo*, the composition may be administered orally or parenterally. Examples of the parenteral administration include an intravenous injection (intravenous administration), an intramuscular injection (intramuscular administration), percutaneous administration, subcutaneous administration, intracutaneous administration, enteral administration, rectal administration, transvaginal administration, transnasal administration, lung administration, intraperitoneal administration, and topical administration.

The dosage form of the composition of the present invention is not particularly limited and is determined as appropriate, for example, in accordance with the administration form. Examples of the dosage form include a liquid and a solid.

The composition of the present invention may also include, for example, an additive as necessary. The additive is preferably a pharmaceutically acceptable additive or a pharmaceutically acceptable carrier.

A method for manufacturing the composition of the present invention (hereinafter, also referred to as a "manufacturing method" ) includes a treatment step of treating megakaryocytes or a culture thereof. In the manufacturing method of the present invention, the treatment target in the treatment step is megakaryocytes or a culture thereof. Accordingly, the manufacturing method of the present invention may include a megakaryocyte-inducing step of inducing the megakaryocytes from cells that are less differentiated than megakaryocytes and/or a production step of producing the culture of megakaryocytes that are performed prior to the treatment step.

In the megakaryocyte-inducing step, there is no particular limitation on a method for inducing the megakaryocytes, and the megakaryocytes can be induced using a known induction method. Specifically, regarding the method for inducing the megakaryocytes, the methods for inducing immortalized megakaryocytes disclosed in WO 2011/034073 (US 2012/0238023A), WO 2012/157586 (US 2014/0127815A), WO 2014/123242 (US 2016/0002599A), and the like; and the method for inducing megakaryocytes described in Reference 6 below can be referred to, for example, and these methods are incorporated by reference herein and constitute part of this specification. Specifically, in the megakaryocyte-inducing step, the oncogene and the polycomb gene may be, for example, forcedly expressed in the cells that are less differentiated than megakaryocytes. Thus, in the megakaryocyte-inducing step, for example, immortalized megakaryocytes that indefinitely proliferate can be obtained. Furthermore, for example, stopping the forced expression in the immortalized megakaryocytes makes it possible to induce multinucleated megakaryocytes from the immortalized megakaryocytes and enable production of platelets. Also, in the megakaryocyte-inducing step, for example, the apoptosis inhibitor gene may be forcedly expressed in the megakaryocyte progenitor cells. Thus, in the megakaryocyte-inducing step, the immortalized megakaryocytes can be obtained. Furthermore, for example, stopping the forced expression in the immortalized megakaryocytes makes it possible to induce multinucleated megakaryocytes from the immortalized megakaryocytes and enable production of platelets.

Reference 6: Ann-Kathrin Borger et.al., "Generation of HLA-Universal iPSC-Derived Megakaryocytes and Platelets for Survival Under Refractoriness Conditions", Mol. Med., 2016, vol. 22, pages 274-288

In the megakaryocyte-inducing step, for example, the oncogene, the polycomb gene, and the apoptosis inhibitor gene may be forcedly expressed. In this case, the oncogene, the polycomb gene, and the apoptosis inhibitor gene may be forcedly expressed simultaneously or separately. Specifically, in the megakaryocyte-inducing step, a procedure may be employed in which the oncogene and the polycomb gene are forcedly expressed, this forced expression is then stopped, and subsequently, the apoptosis inhibitor gene is forcedly expressed, or a procedure may be employed in which the oncogene, the polycomb gene, and the apoptosis inhibitor gene are forcedly expressed, or a procedure may be employed in which the oncogene and the polycomb gene are forcedly expressed, and then the apoptosis inhibitor gene is further expressed. Thus, in the megakaryocyte-inducing step, immortalized megakaryocytes can be obtained. Furthermore, for example, stopping the forced expression in the immortalized megakaryocytes makes it possible to induce multinucleated megakaryocytes from the immortalized megakaryocytes and enable production of platelets.

It is preferable that the megakaryocyte-inducing step includes, for example, a first expression step of forcedly expressing an oncogene and a polycomb gene in the cells that are less differentiated than megakaryocytes, a second expression step of forcedly expressing an apoptosis inhibitor gene such as the Bcl-xL gene in the less differentiated cells, and a stopping step of stopping all of the forced gene expression, as the introduction efficiency of the genes can be improved.

The forced expression of each gene can be carried out and stopped using, for example, known methods such as the methods disclosed in WO 2011/034073, WO 2012/157586, WO 2014/123242, and Reference 7 below, or equivalent methods, and these methods are incorporated herein and constitute part of this specification. Specifically, the forced expression of each gene can be carried out and stopped using, for example, a drug-responsive gene expression-inducing system. Examples of the gene expression-inducing system include a Tet-on^{®} system, and a Tet-off^{®} system. In the case where the Tet-on^{®} system is used, for example, the culturing is performed in the presence of a drug such as tetracycline or doxycycline that induces gene expression, in the step of carrying out the forced expression, and the culture is performed in the absence of the drug in the step of stopping the forced expression.

Reference 7: Nakamura S et al, "Expandable megakaryocyte cell lines enable clinically applicable generation of platelets from human induced pluripotent stem cells.", Cell Stem Cell, 2014, vol. 14, No.4, pages 535-548

Next, in the production step, the culture of megakaryocytes is produced. The production step can be carried out by, for example, culturing the megakaryocytes in the presence of a culture medium. The megakaryocytes may be cultured, for example, on feeder cells or without feeder cells. The megakaryocytes can be cultured in, for example, suspension culture, and can thus be cultured without the feeder cells. The culture of megakaryocytes includes, for example, the platelets.

There is no particular limitation on the megakaryocyte culturing conditions, and common megakaryocyte culturing conditions can be employed. Specifically, the culture temperature is, for example, about 35°C to about 42°C, about 36°C to about 40°C, or about 37°C to about 39°C. The CO₂ concentration is, for example, about 5% to about 15%. The O₂ concentration is, for example, about 15% to about 25%, or about 20%. The culture period is not particularly limited, and is, for example, about 1 day to about 2 weeks, or about 4 days to about 8 days.

There is no particular limitation on the culture medium, and examples thereof include known culture media suited to produce platelets from the megakaryocytes, and equivalent culture media. Specifically, the culture medium can be prepared, for example, using, as a basal culture medium, a culture medium that is used to culture animal cells. Examples of the basal culture medium include single culture media such as an IMDM culture medium, a Medium 199 culture medium, an Eagle's Minimum Essential Medium (EMEM) culture medium, an αMEM culture medium, a Dulbecco's modified Eagle's Medium (DMEM), a Ham's F12 culture medium, an RPMI1640 culture medium, a Fischer's culture medium, and a Neurobasal^{®} Medium (manufactured by Thermo Fisher Scientific), and culture media obtained by mixing these culture media. For example, the culture medium may include serum or plasma, or may be a serum-free culture medium that does not include serum or plasma. The source of the serum and plasma is preferably the same as the source of the megakaryocytes. Specifically, in the case where the megakaryocytes are derived from a human, both the serum and the plasma are preferably derived from a human.

The culture medium may include, for example, other components. There is no particular limitation on the other components, and examples thereof include albumin, insulin, transferrin, selenium, fatty acids, microelements, 2-mercaptoethanol, thiolglycerol, monothioglycerol (MTG), lipids, amino acids (e.g., L-glutamine), ascorbic acid, heparin, non-essential amino acids, vitamins, growth factors, low-molecular-weight compounds, antibiotics, antioxidants, pyruvic acid, buffers, inorganic salts, and cytokines. The other components may be used, for example, alone or in combination of two or more. The cytokines are, for example, substances that promote differentiation of blood cells, and specific examples thereof include a vascular endothelial growth factor (VEGF), thrombopoietin (TPO), various TPO-like agonists, a stem cell factor (SCF), an ITS (insulin-transferrin-selenite) supplement, an ADAM inhibitor, an FLT inhibitor, a WNT inhibitor, a ROCK inhibitor, and an aryl hydrocarbon receptor (AhR) inhibitor. The culture medium is preferably, for example, an IMDM medium that includes serum, insulin, transferrin, serine, thiolglycerol, ascorbic acid, and TPO. For example, the culture medium may further include SCF, and may further include heparin. There is no particular limitation on the concentrations of the other components. The concentration of the TPO is, for example, about 10 ng/ml to about 200 ng/ml, or about 50 ng/ml to about 100 ng/ml. The concentration of the SCF is, for example, about 10 ng/ml to about 200 ng/ml, or about 50 ng/ml. The concentration of the heparin is, for example, about 10 U/ml to about 100 U/ml, or about 25 U/ml. The culture medium may further include, for example, a phorbol ester (e.g., phorbol-12-myristate-13-acetate; PMA).

Next, in the treatment step, the megakaryocytes or a culture thereof is treated. In the treatment step, biomacromolecules such as proteins are extracted by, for example, disrupting the cell membranes of the megakaryocytes or the cell membranes of cells contained in the culture of megakaryocytes. Specifically, there is no particular limitation on the treatment in the treatment step, and examples of the treatment include: treatment for changing the density of the cellular component, such as concentrating; extraction treatment for extracting the cellular component, such as drying, freezing, freeze-drying, solvent treatment, surfactant treatment, enzyme treatment, protein-fraction extraction treatment, and ultrasonic treatment; and disruption treatment such as grinding and pulverizing. One type or two or more types of treatment may be carried out in the treatment step. The treatment may be carried out once or twice or more in the treatment step.

The concentrating can be carried out by, for example, centrifuging the megakaryocytes or the culture thereof. The concentrating can be carried out, for example, under conditions where cells or platelets are precipitated. The drying can be carried out by, for example, drying the megakaryocytes or the culture thereof using a dry spray, a drum dryer, or the like. The freeze-drying can be carried out using, for example, a freeze-dryer. In the solvent treatment, the solvent is, for example, an organic solvent such as phenol or chloroform, or an aqueous solvent such as water, a physiological saline solution, or a buffer solution. In the case where an aqueous solvent is used as the solvent, it is preferable to carry out the solvent treatment together with, for example, surfactant treatment, enzyme treatment, and/or ultrasonic treatment, which will be described later. The solvent treatment can be carried out by, for example, mixing the megakaryocytes or the culture thereof with the solvent. In the surfactant treatment, examples of the surfactant include: ionic surfactants such as sodium lauryl sulfate; nonionic surfactants such as NP-40, Triton X-100, Tween 20, and n-dodecyl-β-D-maltopyranoside; and zwitterionic surfactants such as CHAPS. The concentration of the surfactant is, for example, a concentration at which the cell membranes of the megakaryocytes or the cell membranes of cellular components in the culture of megakaryocytes can be disrupted. The surfactant treatment can be carried out by, for example, bringing the megakaryocytes or the culture thereof into contact with the surfactant in the presence of an aqueous solvent. The contact with the surfactant is carried out, for example, at about 0°C to about 10°C. Examples of the aqueous solvent include water, a physiological saline solution, and a buffer solution. Examples of an enzyme used in the enzyme treatment include peptidases and proteases. The enzyme treatment can be carried out by, for example, bringing the megakaryocytes or the culture thereof into contact with the enzyme in the presence of an aqueous solvent. The enzyme treatment is carried out under conditions where, for example, the enzyme exhibits activity. The protein-fraction extraction treatment can be carried out by, for example, performing application of osmotic shock, freeze-thaw processing, or the like on the megakaryocytes or the culture thereof. The ultrasonic treatment can be carried out using, for example, an ultrasonic generator. The ultrasonic treatment is carried out, for example, under conditions where cells are disrupted.

The treatment conditions and the treatment period of each treatment can be determined as appropriate, for example, in accordance with the treatment type. In the treatment step, the concentration of the total protein in the treated product can be adjusted by, for example, adjusting the amount of the aqueous solvent.

The megakaryocytes and the culture thereof may be manipulated (pretreated) prior to the treatment. In this case, a culture itself obtained by culturing the megakaryocytes, or a product obtained by manipulating the mixture may be used as the culture of megakaryocytes. Examples of the culture manipulation include removal of a liquid fraction, extraction of a cellular component fraction, and a change in the composition of the cellular components that include platelets. Examples of the change in the composition of the cellular components include removal of cells and/or platelets in the mixture, extraction of cells and/or platelets in the mixture, and addition of cells and/or platelets to the mixture.

In the case where the manufacturing method of the present invention includes the pretreatment, the manufacturing method of the present invention may include a removal step of removing platelets from the megakaryocytes or the culture of megakaryocytes. In this case, in the treatment step, the treatment is carried out using, as the megakaryocytes or the culture thereof, the megakaryocytes or the culture thereof from which platelets have been removed, or the removed platelets. After releasing platelets, for example, the megakaryocytes contain a large amount of bFGF. Accordingly, with the manufacturing method of the present invention, a composition containing a large amount of bFGF can be manufactured by, for example, removing the platelets. The platelets can be separated from the other cellular fraction by removing the platelets. Accordingly, the removal step can also be referred to as, for example, a "platelet separation step" or "step of separating platelets from the other cellular components". In the removal step, platelets can be separated from the culture of megakaryocytes using, for example, a known method such as the method disclosed in WO 2017/065280 (US 2018/282697A), or equivalent methods, and these methods are incorporated herein and constitute part of this specification.

The platelet removal ratio (separation ratio) in the removal step is, for example, 60% or more, 70% or more, 80% or more, 85% or more, 90% or more, 95% or more, 96% or more, 97% or more, 98% or more, or 99% or more. The platelet removal ratio is, for example, 60 to 90%.

The manufacturing method of the present invention may include a preservation step of preserving the megakaryocytes or the culture thereof, the megakaryocytes or the culture thereof from which platelets have been removed, or removed platelets. Examples of the preservation in the preservation step include refrigerated preservation (about 1°C to about 10°C) and frozen preservation (about -200°C to about -4°C). There is no particular limitation on the preservation period in the preservation step. It is preferable to employ the frozen preservation in the preservation step, for example, because it also serves as the freezing in the treatment step.

The manufacturing method of the present invention can be carried out, for example, as follows. However, the present invention is not limited to the example below. First, the culture medium containing the megakaryocytes or the culture thereof is subjected to centrifugal concentration, and thus the cellular components are concentrated. The centrifugation is carried out under conditions where, for example, the cellular components are precipitated. Specifically, the centrifugation can be carried out, for example, at 1000 to 3000×g for 5 to 20 minutes. Next, after the centrifugation, the precipitate is collected, and quick freezing is performed to freeze the obtained precipitate. The freezing can be carried out by, for example, bringing the precipitate into contact with a liquefied gas such as liquid nitrogen. Furthermore, the surfactant treatment is carried out by thawing the frozen precipitate in an aqueous solvent containing the surfactant. The obtained solution is centrifuged to precipitate unwanted substances. The centrifugation is carried out under conditions where, for example, unwanted substances such as cell membranes are precipitated. Specifically, the centrifugation can be carried out, for example, at 10000 to 20000×g for 3 to 10 minutes. After the centrifugation, proteins are present in the supernatant, and therefore, the treated product can be obtained by collecting the supernatant as a protein fraction.

The composition of the present invention can be used as, for example, an osteoblast differentiation-promoting composition as described later. The descriptions of a cell proliferation-promoting composition and an osteoblast differentiation-promoting composition, which will be described later, can be applied to the method for using the present invention. Also, it is assumed that the composition of the present invention can be favorably used for, for example, jawbone defect caused by jawbone tumor, a cyst, osteonecrosis, an external wound, or a bone disease (e.g., ectodermal dysplasia or cheilognathopalatoschisis); periodontal diseases or alveolar bone defects such as alveolar bone defects associated with the periodontal diseases or alveolar bone defects caused by caries or external trauma; osteoporosis such as primary osteoporosis (e.g., postmenopausal osteoporosis or senile osteoporosis) or secondary osteoporosis (e.g., osteogenesis imperfecta); refractory bone fracture caused by hyperthyroidism, Cushing syndrome, hypogonadism, or an external wound; costal bone fixation after open chest surgery; bone defect caused by craniotomy; systemic bone defect caused by tumor excision or cyst excision; and the like.

### Osteoblast Differentiation-Promoting Composition

An osteoblast differentiation-promoting composition of the present invention includes the composition of the present invention as described above. The differentiation-promoting composition of the present invention is characterized by including the composition of the present invention, and there is no particular limitation on configurations and conditions other than this. The differentiation-promoting composition of the present invention is capable of promoting differentiation to osteoblasts, particularly differentiation of mesenchymal stem cells to osteoblasts. The description of the composition of the present invention can be applied to the differentiation-promoting composition of the present invention.

The differentiation-promoting composition of the present invention may be used *in vitro* or *in vivo.*

In the case where the differentiation-promoting composition of the present invention is used *in vitro,* examples of the administration subject include cells, tissues, and organs, and examples of the cells include cells collected from a living organism, and cultured cells.

In the case where the differentiation-promoting composition of the present invention is used *in vivo,* examples of the administration subject include a human and a non-human animal other than a human. Examples of the non-human animal include a mouse, a rat, a rabbit, a dog, a sheep, a horse, a cat, a goat, a monkey, and a guinea pig.

There is no particular limitation on the use conditions (administration conditions) of the differentiation-promoting composition of the present invention, and the administration form, the timing of administration, the dosage amount, and the like can be determined as appropriate, for example, in accordance with the type of administration subject. The description of the composition of the present invention can be applied to the administration conditions of the differentiation-promoting composition of the present invention.

In the case where the differentiation-promoting composition of the present invention is used *in vitro,* the differentiation-promoting composition of the present invention can be added to, for example, a culture medium of target progenitor cells. The differentiation-promoting composition of the present invention may be added to a maintenance culture medium used to maintain the progenitor cells, or to a differentiation culture medium used to differentiate the progenitor cells to the osteoblasts, or to both the maintenance culture medium and the differentiation culture medium. With the differentiation-promoting composition of the present invention, by maintaining the progenitor cells in, for example, a maintenance culture medium containing the differentiation-promoting composition, it is possible to also promote differentiation when the progenitor cells subsequently differentiate to osteoblasts. The final concentration of the total protein derived from the differentiation-promoting composition of the present invention in the maintenance culture medium is, for example, 10 to 1000 µg/ml, 10 to 500 µg/ml, or 10 to 300 µg/ml. The final concentration of the total protein derived from the differentiation-promoting composition of the present invention in the differentiation culture medium is, for example, 10 to 1000 µg/ml, 10 to 500 µg/ml, or 10 to 300 µg/ml.

An effect of promoting differentiation of progenitor cells to osteoblasts can be evaluated depending on, for example, whether or not differentiation of mesenchymal stem cells to osteoblasts can be promoted in the presence of a test substance compared with the mesenchymal stem cells in the absence of the test substance (control group). Specifically, the effect of promoting differentiation to osteoblasts can be evaluated using, as an indicator, the calcium ion concentration in the culture medium of a cell group produced by inducing differentiation of the mesenchymal stem cells to osteoblasts. A method for inducing the differentiation of mesenchymal stem cells to osteoblasts and a method for measuring the calcium ion concentration can be carried out in conformity with Example 1(6), which will be described later. In the evaluation above, the test substance can be evaluated as having the effect of promoting differentiation to osteoblasts when the calcium concentration in the presence of the test substance is, for example, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, or 5% or less of the calcium concentration in the absence of the test substance.

In the assay for induction of differentiation of mesenchymal stem cells to osteoblasts, the differentiation-promoting composition of the present invention has, for example, osteoblast differentiation-promoting activity that suppresses the calcium concentration to 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, or 5% or less. The assay for induction of differentiation of mesenchymal stem cells to osteoblasts can be carried out in conformity with Example 1(6) will be described later.

In the case where the differentiation-promoting composition of the present invention is used *in vivo,* the dosage amount of the differentiation-promoting composition can be determined as appropriate, for example, in accordance with the type, condition, and age of the administration subject, the administration method, and the like. Specifically, in the case of administration to a human, the sum of the amounts of all the proteins derived from the differentiation-promoting composition is not particularly limited and the dosage amount of the differentiation-promoting composition per day can be determined as appropriate, for example, in accordance with the application thereof. The administration frequency per day is, for example, 1 to 5 times, 1 to 3 times, or once or twice.

There is no particular limitation on the administration form of the differentiation-promoting composition of the present invention. In the case where the differentiation-promoting composition of the present invention is administered *in vivo,* the differentiation-promoting composition may be administered orally or parenterally. Examples of the parenteral administration include an intravenous injection (intravenous administration), an intramuscular injection (intramuscular administration), percutaneous administration, subcutaneous administration, intracutaneous administration, enteral administration, rectal administration, transvaginal administration, transnasal administration, lung administration, intraperitoneal administration, and topical administration.

The dosage form of the differentiation-promoting composition of the present invention is not particularly limited and is determined as appropriate, for example, in accordance with the administration form. Examples of the dosage form include a liquid and a solid.

The differentiation-promoting composition of the present invention may also include, for example, an additive as necessary. The additive is preferably a pharmaceutically acceptable additive or a pharmaceutically acceptable carrier.

### Osteogenic Kit

An osteogenic kit of the present invention includes an osteogenic composition and a scaffold material for osteogenesis, and the osteogenic composition is the osteogenic composition of the present invention. The kit of the present invention is characterized by including the osteogenic composition of the present invention, and there is no particular limitation on configurations and conditions other than this. With the kit of the present invention, osteogenesis can be easily induced by using the composition of the present invention and the scaffold material in combination. The descriptions of the composition and the differentiation-promoting composition of the present invention can be applied to the kit of the present invention.

In the kit of the present invention, a scaffold material used to, for example, regenerate bones or cartilages can be used as the scaffold material. The scaffold material can also be referred to as, for example, a material that enables attachment, proliferation, and/or differentiation of osteocytes or osteoblasts, or progenitor cells thereof. The scaffold material is, for example, a structure (construct) having a three-dimensional hollow structure or porous structure, and specific examples thereof include a porous body, a porous support, a three-dimensional porous structure (construct), a gel (hydrogel), and a spongy construct.

It is preferable that the scaffold material has biocompatibility and/or biodegradability because the scaffold material is, for example, retained together with the composition of the present invention in a living organism. The term "biocompatibility" means that, for example, the scaffold material has compatibility with a tissue and/or organ in a living organism, and thus a foreign-body reaction, rejection, and the like do not occur. The term "biodegradability" means that, for example, constituent components of the scaffold material can be degraded in a living organism.

Examples of the constituent components of the scaffold material include: proteins such as collagen, gelatin, albumin, keratin, fibrin, and fibroin; polysaccharides such as agarose, dextran, carboxymethyl cellulose, xanthan gum, chitosan, chondroitin sulfate, heparin, hyaluronic acid, and alginic acid; polyglycolic acid (PGA), polylactic acid (PLA), copolymers of polyglycolic acid and polylactic acid, polyhydroxybutyrate, polydioxanone, polyethylene glycol (PEG), polycaprolactone, polybutylene succinate, calcium phosphate (e.g., β-tricalcium phosphate), calcium carbonate, hydroxyapatite, polyetherketone, and polyetheretherketone. The constituent components may be used alone or in combination of two or more.

The scaffold material can be prepared by, for example, cross-linking the constituent components of the scaffold material or forming a complex of the constituent components.

Examples of the scaffold material include NEOBONE (made of hydroxyapatite; manufactured by Aimdec MMT Co., Ltd.), APACERAM^{®} (made of hydroxyapatite; manufactured by HOYA Corporation), Bonarc (made of collagen/octacalcium phosphate; manufactured by TOYOBO Co., Ltd.), OSferion (made of βTCP; manufactured by Olympus Terumo Biomaterials Corp.), Cytrans Granules (made of carbonate apatite; manufactured by GC), and ReFit (made of collagen/apatite; manufactured by HOYA Corporation).

In the kit of the present invention, the composition and the scaffold material may exist independently of each other or may be integrated. In the latter case, the kit of the present invention can also be referred to as an "osteogenic scaffold material". In the case where the composition and the scaffold material are integrated, the composition may adsorb onto the surface of the scaffold material, or may be included in the scaffold material.

The kit of the present invention may also include, for example, a physiologically active substance in addition to the composition. The physiologically active substance promotes, for example, osteogenesis, or differentiation of osteoblasts or osteocytes. Examples of the physiologically active substance include a vascular endothelial growth factor (VEGF), a platelet-derived growth factor (PDGF), an epidermal growth factor (EGF), a fibroblast growth factor (FGF), a hepatocyte growth factor (HGF), an insulin-like growth factor (IGF), a brain-derived neurotrophic factor (BDNF), a growth differentiation factor-5 (GDF5), an erythropoiesis-stimulating factor (EPO), a transforming growth factor (TGF), and a bone morphogenetic protein (BMP). The physiologically active substances may be used alone or in combination of two or more.

The kit of the present invention can be used by, for example, transplanting (burying, embedding) the composition and the scaffold material in a site of the administration subject where induction of osteogenesis is desired. Specifically, in the case where the composition and the scaffold material are independent of each other in the kit of the present invention, the kit can be used by impregnating the scaffold material with the composition, and then transplanting the scaffold material. Also, in the case where the composition and the scaffold material are independent of each other in the kit of the present invention, the kit may also be used by transplanting the scaffold material and then introducing the composition of the present invention into the transplantation site. Also, in the case where the composition and the scaffold material are integrated in the kit of the present invention, the kit can be used by transplanting the scaffold material.

In the kit of the present invention, the content of the composition can be set, for example, in accordance with the dosage amount of the composition of the present invention.

### Immunocyte Activation Inhibitory Composition

Another aspect of the present invention provides a composition having immunocyte activation inhibitory activity derived from cells. In this case, the immunocyte activation inhibitory composition of the present invention (hereinafter, also referred to as an "activation inhibitory composition" ) includes a treated product obtained by treating megakaryocytes or treating a culture thereof. The activation inhibitory composition of the present invention is characterized by including a treated product obtained by treating megakaryocytes or treating a culture thereof, and there is no particular limitation on configurations and conditions other than this. The activation inhibitory composition of the present invention can be used to, for example, provide a composition having immunocyte activation inhibitory activity derived from cells.

The descriptions of the composition, the differentiation-promoting composition, and the osteogenic kit of the present invention can be applied to the activation inhibitory composition of the present invention.

The activation inhibitory composition of the present invention is capable of, for example, inhibiting production of inflammatory cytokines in immunocytes. Accordingly, the activation inhibitory composition of the present invention can also be referred to as, for example, a "composition for inhibiting production of inflammatory cytokines in immunocytes".

In the present invention, the term "immunocyte activation" may be used in a sense that expression of an activation marker is induced in immunocytes or a sense that expression of cytokines such as inflammatory cytokines is induced. Examples of the inflammatory cytokines include TNF-α, IL-1β, IL-6, and IFN-γ.

The immunocyte activation inhibitory effect can be evaluated depending on, for example, whether or not the concentration of inflammatory cytokines in the culture medium after culturing immunocytes in the presence of a test substance under conditions where the immunocytes are activated can be reduced compared with the case of culturing immunocytes in the absence of the test substance (control group). Specifically, the immunocyte activation inhibitory effect can be evaluated based on the rate of a reduction in the amount of produced inflammatory cytokines. The type of immunocyte, the activation conditions, and the amount of produced inflammatory cytokines can be measured in conformity with Example 5, which will be described later. In the evaluation above, the test substance can be evaluated as having the immunocyte activation inhibitory effect when the rate of a reduction in the amount of at least one of produced cytokines is, for example, 10% or more, 20% or more, 30% or more, 40% or more, 50% or more, 60% or more, 70% or more, 75% or more, 80% or more, 85% or more, 90% or more, 95% or more, or 99% or more.

In the immunocyte activation assay, the activation inhibitory composition of the present invention has, for example, an immunocyte activation inhibitory effect that suppresses the concentration of at least one inflammatory cytokine to 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, or 5% or less, as compared with that of the control group. The immunocyte activation assay can be carried out in conformity with Example 5, which will be described later.

In the present invention, examples of the immunocytes include: lymphocytes such as T cells; and macrophages such as inflammatory macrophages.

The activation inhibitory composition of the present invention may be used *in vitro* or *in vivo.*

In the case where the activation inhibitory composition of the present invention is used *in vitro,* examples of the administration subject include cells, tissues, and organs, and examples of the cells include cells collected from a living organism, and cultured cells.

In the case where the activation inhibitory composition of the present invention is used *in vivo,* examples of the administration subject include a human and a non-human animal other than a human. Examples of the non-human animal include a mouse, a rat, a rabbit, a dog, a sheep, a horse, a cat, a goat, a monkey, and a guinea pig.

There is no particular limitation on the use conditions (administration conditions) of the activation inhibitory composition of the present invention, and the administration form, the timing of administration, the dosage amount, and the like can be determined as appropriate, for example, in accordance with the type of administration subject.

In the case where the activation inhibitory composition of the present invention is used *in vitro,* the activation inhibitory composition of the present invention can be added to, for example, a culture medium of target cells. In the case where the activation inhibitory composition of the present invention is used *in vitro,* the final concentration of the total protein derived from the activation inhibitory composition of the present invention in the culture medium is, for example, 10 to 1000 µg/ml, 10 to 500 µg/ml, or 10 to 300 µg/ml.

In the case where the activation inhibitory composition of the present invention is used *in vivo,* the dosage amount of the activation inhibitory composition can be determined as appropriate, for example, in accordance with the type, condition, and age of the administration subject, the administration method, and the like. Specifically, in the case of administration to a human, the sum of the amounts of all the proteins derived from the activation inhibitory composition is not particularly limited and the dosage amount of the activation inhibitory composition per day can be determined as appropriate, for example, in accordance with the application thereof. The administration frequency per day is, for example, 1 to 5 times, 1 to 3 times, or once or twice.

The description of the composition of the present invention can be applied to the administration conditions and the dosage form of the activation inhibitory composition of the present invention.

### Anti-Inflammatory Composition

Another aspect of the present invention provides a composition having cell-derived anti-inflammatory activity. In this case, an anti-inflammatory composition of the present invention includes a treated product obtained by treating megakaryocytes or treating a culture thereof. The anti-inflammatory composition of the present invention is characterized by including a treated product obtained by treating megakaryocytes or treating a culture thereof, and there is no particular limitation on configurations and conditions other than this. The anti-inflammatory composition of the present invention can be used to, for example, provide a composition capable of inhibiting inflammation. The descriptions of the composition, the differentiation-promoting composition, the osteogenic kit, and the activation inhibitory composition of the present invention can be applied to the anti-inflammatory composition of the present invention.

In the present invention, the term "anti-inflammatory" means having an ability to inhibit inflammation, which can be investigated, for example, using, as an indicator, inhibition of the induction of expression of inflammatory cytokines or inhibition of the production of inflammatory cytokines.

The anti-inflammatory composition of the present invention may be used *in vitro* or *in vivo.*

The descriptions of the composition and the immunocyte activation inhibitory composition of the present invention can be applied to the administration subject, the administration conditions, and the dosage form of the anti-inflammatory composition of the present invention.

### Osteogenic Method

In an osteogenic method of the present invention, the osteogenic composition of the present invention is used. The osteogenic method of the present invention is characterized by using the composition of the present invention, and there is no particular limitation on configurations and conditions other than this. With the osteogenic method of the present invention, it is possible to promote, for example, differentiation to osteoblasts, particularly differentiation of mesenchymal stem cells to osteoblasts, and thus induce osteogenesis. The descriptions of the composition, the differentiation-promoting composition, and the osteogenic kit of the present invention can be applied to the osteogenic method of the present invention.

The osteogenic method of the present invention includes, for example, an administration step of administering the osteogenic composition of the present invention to an administration subject. In the case where osteogenesis is induced *in vivo* using the osteogenic method of the present invention, the composition of the present invention can be administered to a site where induction of osteogenesis is desired. The descriptions of the administration subject and the administration conditions of the composition of the present invention can be applied to the administration subject and the administration conditions in the osteogenic method of the present invention.

In an osteogenic method of the present invention, the osteogenic kit of the present invention may also be used as the osteogenic composition of the present invention.

### Osteoblast Differentiation-Promoting Method

In an osteoblast differentiation-promoting method of the present invention (also referred to as a "differentiation-promoting method" hereinafter), the cell differentiation-promoting composition of the present invention is used. The differentiation-promoting method of the present invention is characterized by using the differentiation-promoting composition of the present invention, and there is no particular limitation on configurations and conditions other than this. With the differentiation-promoting method of the present invention, it is possible to promote differentiation to osteoblasts, particularly differentiation of mesenchymal stem cells to osteoblasts. The descriptions of the composition, the differentiation-promoting composition, the osteogenic kit, and the osteogenic method of the present invention can be applied to the differentiation-promoting method of the present invention.

The differentiation-promoting method of the present invention may be carried out, for example, *in vitro* or *in vivo.* The differentiation-promoting method of the present invention includes, for example, an administration step of administering the differentiation-promoting composition of the present invention to an administration subject.

In the case where the differentiation-promoting method of the present invention is carried out *in vitro,* the differentiation-promoting method of the present invention includes, for example, a differentiation step of differentiating progenitor cells to osteoblasts in the presence of the differentiation-promoting composition. The differentiation-promoting method of the present invention may include a maintenance step of carrying out maintenance culture of progenitor cells in the presence of the differentiation-promoting composition prior to the differentiation step. For example, the descriptions of the administration subject and the administration conditions of the differentiation-promoting composition of the present invention can be applied to the administration subject and the administration conditions of the differentiation-promoting composition of the present invention.

### Immunocyte Activation-Inhibiting Method

Another aspect of the present invention provides an immunocyte activation-inhibiting method. In the immunocyte activation-inhibiting method of the present invention (hereinafter, also referred to as an "activation-inhibiting method" ), the immunocyte activation-inhibiting composition of the present invention is used. The activation-inhibiting method of the present invention is characterized by using the activation inhibitory composition of the present invention, and there is no particular limitation on configurations and conditions other than this. With the activation-inhibiting method of the present invention, it is possible to inhibit immunocyte activation. The descriptions of the composition, the differentiation-promoting composition, the osteogenic kit, the activation inhibitory composition, the anti-inflammatory composition, the osteogenic method, and the differentiation-promoting method of the present invention can be applied to the activation-inhibiting method of the present invention.

The activation-inhibiting method of the present invention may be carried out, for example, *in vitro* or *in vivo.* The activation-inhibiting method of the present invention includes, for example, an administration step of administering the activation inhibitory composition of the present invention to an administration subject.

In the case where the activation-inhibiting method of the present invention is carried out *in vitro,* the activation-inhibiting method of the present invention includes, for example, a culture step of culturing immunocytes in the presence of the activation inhibitory composition. For example, the descriptions of the administration subject, the administration conditions, and the dosage form of the activation inhibitory composition of the present invention can be applied to the administration subject, the administration conditions, and the dosage form of the activation inhibitory composition of the present invention.

### Inflammation-Inhibiting Method

Another aspect of the present invention provides a method for inhibiting inflammation. In the inflammation-inhibiting method of the present invention (hereinafter, also referred to as an "inhibiting method" ), the anti-inflammatory composition of the present invention is used. The inflammation-inhibiting method of the present invention is characterized by using the anti-inflammatory composition of the present invention, and there is no particular limitation on configurations and conditions other than this. With the inflammation-inhibiting method of the present invention, it is possible to inhibit inflammation. The descriptions of the composition, the differentiation-promoting composition, the osteogenic kit, the activation inhibitory composition, the anti-inflammatory composition, the osteogenic method, the differentiation-promoting method, and the activation-inhibiting method of the present invention can be applied to the inflammation-inhibiting method of the present invention.

The inflammation-inhibiting method of the present invention may be carried out, for example, *in vitro* or *in vivo.* The inflammation-inhibiting method of the present invention includes, for example, an administration step of administering the anti-inflammatory composition of the present invention to an administration subject.

In the case where the inflammation-inhibiting method of the present invention is carried out *in vitro,* the inflammation-inhibiting method of the present invention includes, for example, a culture step of culturing the administration subject in the presence of the anti-inflammatory composition. For example, the descriptions of the administration subject, the administration conditions, and the dosage form of the anti-inflammatory composition of the present invention can be applied to the administration subject, the administration conditions, and the dosage form of the anti-inflammatory composition of the present invention.

### Use of Composition

The present invention relates to a composition for use to induce osteogenesis or use of the composition, the composition including, as an active component, a treated product obtained by treating megakaryocytes or a culture thereof. The present invention relates to a composition for use to promote differentiation of osteoblasts or use of the composition, the composition including, as an active component, a treated product obtained by treating megakaryocytes or a culture thereof. The present invention relates to a composition for use to inhibit immunocyte activation or use of the composition, the composition including, as an active component, a treated product obtained by treating megakaryocytes or a culture thereof. The present invention relates to a composition for use to inhibit inflammation or the use of the composition, the composition including, as an active component, a treated product obtained by treating megakaryocytes or a culture thereof. The present invention relates to the use of a composition for manufacturing of an osteogenic composition, the composition including, as an active component, a treated product obtained by treating megakaryocytes or a culture thereof. Also, the present invention relates to the use of a composition for manufacturing of an osteoblast differentiation-promoting composition, the composition including, as an active component, a treated product obtained by treating megakaryocytes or a culture thereof. The present invention relates to the use of a composition for manufacturing of an immunocyte activation inhibitory composition, the composition including, as an active component, a treated product obtained by treating megakaryocytes or a culture thereof. The present invention relates to the use of a composition for manufacturing of an anti-inflammatory composition, the composition including, as an active component, a treated product obtained by treating megakaryocytes or a culture thereof. The descriptions of the composition, the differentiation-promoting composition, the osteogenic kit, the activation inhibitory composition, the anti-inflammatory composition, the osteogenic method, the differentiation-promoting method, the activation-inhibiting method, and the inflammation-inhibiting method of the present invention can be applied to the present invention.

### Examples

Hereinafter, the present invention will be described in detail by way of examples, but the present invention is not limited to aspects described in the examples.

### Example 1

The composition of the present invention was manufactured, and it was checked whether the composition included growth factors and growth factor receptors, and had cell proliferation-promoting activity and activity to promote differentiation to osteoblasts.

### (1) Production of Immortalized Megakaryocytes

Immortalized megakaryocytes were produced in accordance with the following procedure.

### (1-1) Preparation of Hematopoietic Progenitor Cells from iPS Cells

Differentiation culture was carried out to differentiate human iPS cells (TKDN SeV2 and NIH5: human fetal dermal fibroblast-derived iPS cells established using Sendai viruses) to blood cells in accordance with the method disclosed in Reference 8 below. Specifically, a human ES/iPS cell colony was cocultured with C3H10T1/2 feeder cells for 14 days in the presence of 20 ng/ml VEGF (manufactured by R&D SYSTEMS), and thus hematopoietic progenitor cells (HPCs) were produced. The coculture was carried out under conditions of 37°C, 20% O₂, and 5% CO₂ (the same conditions apply hereinafter unless otherwise stated).

Reference 8: Takayama N. et al., "Transient activation of c-MYC expression is critical for efficient platelet generation from human induced pluripotent stem cells", J. Exp. Med., 2010, vo. 13, pages 2817-2830

### (1-2) Gene Introduction System

A lentiviral vector system was used as the gene introduction system. A tetracycline-controlled Tet-on^{®} gene expression-inducing system vector was used as the lentiviral vector. The vector was produced by replacing the mOKS cassette of LV-TRE-mOKS-Ubc-tTA-I2G (Reference 9 below) with c-MYC, BMI1 or BCL-xL. The vectors into which c-MYC, BMI1, and BCL-xL had been introduced were referred to as LV-TRE-c-Myc-Ubc-tTA-I2G, LVTRE-BMI1-Ubc-tTA-I2G, and LV-TRE-BCL-xL-Ubc-tTA-I2G, respectively. c-MYC viruses, BMI1 viruses, and BCL-xL viruses were produced by introducing the genes into 293T cells using the lentiviral vectors. The c-MYC gene, the BMI1 gene, and the BCL-xL gene were introduced into the genome sequences of target cells by infecting the target cells with the obtained viruses. These genes that had been stably introduced into the genome sequences could be forcedly expressed by adding doxycycline (clontech #631311) into the culture medium.

Reference 9: Kobayashi, T. et al., "Generation of rat pancreas in mouse by interspecific blastocyst injection of pluripotent stem cells.", Cell, 2010, vol. 142, No. 5, pages 787-799

### (1-3) Infection of hematopoietic progenitor cells with c-MYC viruses and BMI1 viruses

The HPCs obtained using the method of (1-1) above were seeded at 5×10⁴ cells/well on a 6-well plate on which the C3H10T1/2 feeder cells had been seeded in advance, and then c-MYC and BMI1 were forcedly expressed in accordance with the lentiviral method in which the BMI1 viruses and the c-MYC viruses were used. At this time, 6 wells were used for each cell strain. Specifically, the viral particles were added to the culture medium such that MOI (multiplicity of infection) was 20, and the cells were infected with the viruses through spin infection (centrifugation at 32°C and 900 rpm for 60 minutes). The spin infection was carried out twice every 12 hours. A culture medium (hereinafter referred to as a "differentiation culture medium" ) was prepared by adding 50 ng/ml human thrombopoietin (TPO) (R&D SYSTEMS), 50 ng/ml human stem cell factor (SCF) (R&D SYSTEMS), and 2 µg/ml doxycycline (Dox, clontech #631311) to a basal culture medium (IMDM (Iscove's Modified Dulbecco's Medium) (Sigma-Aldrich) containing 15% fetal bovine serum (GIBCO), 1% penicillin-streptomycin-glutamine (GIBCO), 1% insulin, transferrin, selenium solution (ITS-G) (GIBCO), 0.45 mmol/l 1-thioglycerol (Sigma-Aldrich), and 50 µg/ml L-ascorbic acid (Sigma-Aldrich)), and then protamine was added to the differentiation culture medium such that the final concentration was 10 µg/ml. The thus obtained culture medium was used.

### (1-4) Production and Maintenance Culture of Self-Propagating Megakaryocyte Strain

The day when infection with c-MYC viruses and the BMI1 viruses was carried out using the method in (1-3) above was taken as day 0 of the infection. As described below, megakaryocyte self-propagating strains were produced by culturing the HPCs into which the c-MYC gene and the BMI1 gene had been introduced. The c-MYC gene and the BMI1 gene were forcedly expressed by adding DOX to the culture medium such that the DOX concentration was 1 µg/ml.

### - Day 2 to Day 11 of Infection

On day 2 of the infection, the virus-infected blood cells obtained by using the above-mentioned method were collected through pipetting, and were subjected to centrifugation at 1200 rpm for 5 minutes. After the supernatant was removed, the cells were suspended in a new differentiation culture medium and then seeded on new C3H10T1/2 feeder cells (6-well plate). On day 9 of the infection, passage was performed by performing a similar operation. When the cells were re-seeded, the number of cells was counted, and then the cells were seeded at 1×10⁵ cells/2 ml/well on C3H10T1/2 feeder cells (6-well plate).

### - Day 12 to Day 13 of Infection

An operation similar to that on day 2 was performed. The number of cells was counted, and then the cells were seeded at 3×10⁵ cells/10 ml/100-mm dish on C3H10T1/2 feeder cells (100-mm dish).

### - Day 14 of Infection

The virus-infected blood cells were collected, and were reacted with 2 µl of anti-human CD41a-APC antibody (BioLegend), 1 µl of anti-human CD42b-PE antibody (eBioscience), and 1 µl of anti-human CD235ab-pacific blue (BioLegend) antibody per 1.0×10⁵ cells. After the reaction, analysis was performed using FACS Verse^{™} (BD Biosciences). On day 14 of the infection, cells with a CD41a positive rate of 50% or more were taken as megakaryocyte self-propagating strains.

### (1-5) Infection of Megakaryocyte Self-Propagating Strains with BCL-xL Viruses

The BCL-xL gene was introduced into the megakaryocyte self-propagating strains on day 14 of the infection, using the lentiviral method in which BCL-xL viruses were used. The virus particles were added to a culture medium such that MOI was 10, and the cells were infected with the viruses through spin infection (centrifugation at 32°C and 900 rpm for 60 minutes). The BCL-xL gene was forcedly expressed by adding DOX to the culture medium such that the DOX concentration was 1 µg/ml.

### (1-6) Production and Maintenance Culture of Megakaryocyte Immortalized Strain

### - Day 14 to Day 18 of Infection

The megakaryocyte self-propagating strains into which the BCL-xL gene had been introduced using the method in (1-5) above were collected and were subjected to centrifugation at 1200 rpm for 5 minutes. After the centrifugation, the precipitated cells were suspended in a new differentiation culture medium, and were then seeded at 2×10⁵ cells/2 ml/well on C3H10T1/2 feeder cells (6-well plate).

### - Day 18 of Infection: Passage

After the megakaryocyte self-propagating strains into which the BCL-xL gene had been introduced were collected, the number of cells was counted, and then the cells were seeded at 3 × 10⁵ cells/10 ml/100-mm dish.

### - Day 24 of Infection: Passage

After the megakaryocyte self-propagating strains into which the BCL-xL gene had been introduced were collected, the number of cells was counted, and then the cells were seeded at 1× 10⁵ cells/10 ml/100-mm dish. After this, passage was performed every 4 to 7 days in the same manner, and maintenance culture was carried out. Note that when the passage was performed, the cells were suspended in a new differentiation culture medium and were then seeded.

On day 24 of the infection, the megakaryocyte self-propagating strains into which the BCL-xL gene had been introduced were collected, and were analyzed using FACS Verse^{™} after being subjected to immunostaining with 2 µl of anti-human CD41a-APC antibody (BioLegend), 1 µl of anti-human CD42b-PE antibody (eBioscience), and 1 µl of anti-human CD235ab-Pacific Blue (Anti-CD235ab-PB; BioLegend) antibody per 1.0×10⁵ cells. On day 24 of the infection, strains with a CD41a positive rate of 50% or more were taken as immortalized megakaryocyte strains. These cells capable of proliferating for 24 days or more after the infection were taken as immortalized megakaryocyte strains SeV2-MKCL and NIH5-MKCL.

Stationary culture of the obtained SeV2-MKCL and NIH5-MKCL was performed using 10-cm dishes (10 ml/dish). The culture medium was prepared by adding the following components (final concentrations are shown) to IMDM used as a basal culture medium. The culture was carried out under conditions of 37°C and 5% CO₂.
FBS (Sigma #172012 lot. 12E261) 15%
L-Glutamine (Gibco #25030-081) 2 mmol/l
ITS (Gibco #41400-045) 100-fold dilution
MTG (monothioglycerol, Sigma #M6145-25ML) 450 µmol/l
Ascorbic acid (Sigma #A4544) 50 µg/ml
Puromycin (Sigma #P8833-100MG) 2 µg/ml
SCF (Wako Pure Chemical Industries, Ltd. #193-15513) 50 ng/ml
TPO-like agonist 200 ng/ml

### (2) Production of Culture of Megakaryocytes

The forced expression was stopped by culturing the cells in a culture medium containing no DOX. Specifically, the immortalized megakaryocyte strains (SeV2-MKCL and NIH5-MKCL) obtained using the method in (1) above were washed twice using PBS(-), and were then suspended in a platelet-producing culture medium described below. The cell seeding density was set to 1.0×10⁵ cells/ml.

The platelet-producing culture medium was prepared by adding the following components (final concentrations are shown) to IMDM used as a basal culture medium.
human plasma 6%
L-Glutamine (Gibco #25030-081) 4 mmol/l
ITS (Gibco #41400-045) 100-fold dilution
MTG (monothioglycerol, Sigma #M6145-25ML) 450 µmol/l
Ascorbic acid (Sigma #A4544) 50 µg/ml
SCF (Wako Pure Chemical Industries, Ltd. #193-15513) 50 ng/ml
TPO-like agonist 200 ng/ml
ADAM inhibitor 15 µmol/l
GNF351 (Calbiochem #182707) 500 nmol / LY39983 (Chemscene LLC #CS-0096) 500 nmol/l
Urokinase 5 U/ml
Low-molecular-weight heparin (SANOFI, Clexane) 1 U/ml

A culture of megakaryocytes was produced by culturing the cells in the presence of the platelet-producing culture medium for 6 days to cause the cells to produce platelets.

### (3) Manufacturing of Purified Platelets

Platelets were manufactured (purified) from the culture of megakaryocytes obtained in (2) above, in accordance with the following procedure. Note that similar purification was carried out twice.

### (3-1) Concentration of Culture of Megakaryocytes

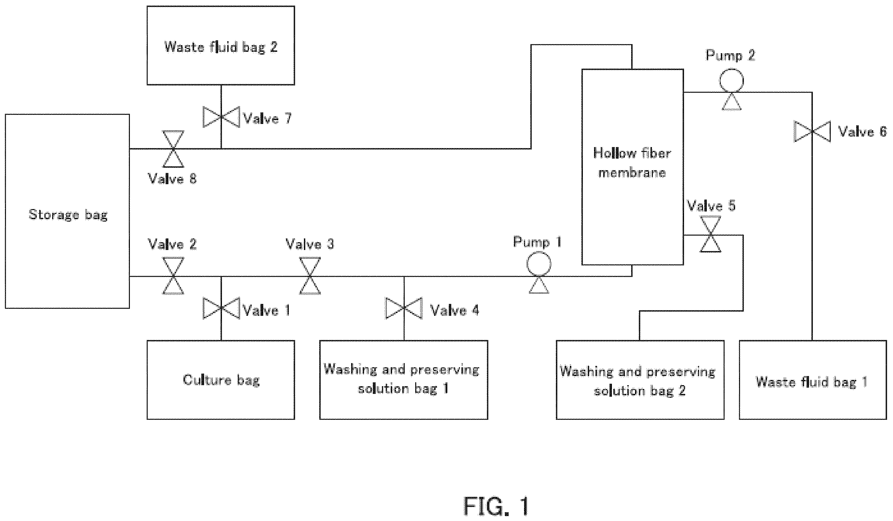

The culture of megakaryocytes obtained in (2) above was introduced into a culture bag. Then, the culture bag was connected to a concentration system as shown in FIG. 1. In FIG. 1, washing and preserving solution bags 1 and 2 contain a washing and preserving solution. A solution obtained by adding 20% ACD and 2.5% human serum albumin to a Bicanate injection (Bicarbon infusion, manufactured by Otsuka Pharmaceutical Co., Ltd.) and adjusting the pH to 7.2 using NaOH was used as the washing and preserving solution. Then, in accordance with Table 1 below, the culture of megakaryocytes was concentrated using a hollow fiber membrane (Plasmaflo OP, manufactured by Asahi Kasei Medical Co., Ltd.), and the obtained concentrate of the culture of megakaryocytes was collected in a storage bag.

**Table 1**

| Procedure | Open valve | Pump 1 (rpm) | Pump 2 (rpm) | Content |
|---|---|---|---|---|
| 1 | 2,3,4 | - | - | 50 mL of washing and preserving solution is introduced from washing and preserving solution bag 1. |
| 2 | 1,3,4 | - | - | 50 mL of washing and preserving solution is introduced from washing and preserving solution bag 1. |
| 3 | 5, 6 | - | 100 | 500 mL of washing and preserving solution is introduced from washing and preserving solution bag 2. Pump 2 is operated for 1 minute. |
| 4 | 4, 7 | 50 | - | 500 mL of washing and preserving solution is introduced from washing and preserving solution bag 1. Pump 1 is operated for 1 minute. |
| 5 | 1,3,6,8 | 100 | 100 | Culture is introduced into hollow fiber membrane. |
| 6 | 2, 3, 6, 8 | 100 | 100 | Concentrate is introduced to storage bag. |
| 7 | 2,3,4 | - | - | 50 mL of washing and preserving solution is introduced from washing and preserving solution bag 1. |
| 8 | 4, 8 | 50 | - | 500 mL of washing and preserving solution is introduced from washing and preserving solution bag 1. |

### (3-2) Centrifugation of Platelets

First, a waste fluid bag of an ACP215 disposable set was replaced with a collecting bag using a sterile tubing welder. A Hicaliq IVH bag (Terumo Corporation HC-B3006A) was used as the collecting bag. Next, 10% amount of an ACD-A solution (manufactured by Terumo Corporation) was added to a concentrate of the culture of megakaryocytes. After the addition, a concentrate to which the ACD-A solution had been added was poured into a cell bag. A Hicaliq IVH bag (Terumo Corporation HC-B3006A) was used as the cell bag.

Next, the cell bag containing the culture to which the ACD-A solution had been added was welded to an ACP215 disposable set, using a sterile tubing welder. Then, ACP215 was started up in a service mode, and the number of rotations was set to 2500 rpm (350×g). The ACP215 was started and the culture in the cell bag was introduced into a separator bowl at about 100 ml/min. A liquid component that flowed out from the separator bowl was collected in the collecting bag. After the entire culture in the cell bag was introduced into the separator bowl, 500 ml of the washing and preserving solution was also introduced into the separator bowl. After the washing and preserving solution was introduced into the separator bowl, the centrifugation was stopped, and the collecting bag containing the collected fluid (collected liquid component containing platelets) was separated using a tube sealer.

The collecting bag containing the collected fluid (containing platelets) was welded to a new ACP215 disposable set using the sterile tubing welder. The ACP215 was started up in a normal mode. The program was set to WPC, and the ACP215 disposable set to which the collecting bag had been welded was set in accordance with the device instructions. Note that the collecting bag containing the collected fluid was arranged on a stand.

Next, the centrifugation speed of the ACP215 was changed to 5000 rpm (1398.8×g), and centrifugation was started. When introduction of the collected fluid into the separator bowl was started, the mode was changed from automatic pouring to manual pouring. Specifically, the collected fluid was introduced into the separator bowl at an introduction speed of about 100 ml/min. After the entire collected fluid was added to the separator bowl, 500 ml of the washing and preserving solution was also added.

### (3-3) Washing of Platelets

In accordance with the program of ACP215, washing was performed using 2000 ml of the washing and preserving solution.

### (3-4) Collection of Platelets

In accordance with the program of ACP215, 200 ml of washed platelets was collected in a platelet product bag.

### (3-5) Separation of Platelets

Platelets were separated from the platelet product bag using the hollow fiber membrane in accordance with a common method, and collected in the collecting bag.

### (4) Manufacturing of Extract

The immortalized megakaryocyte strains obtained using the method in (1) above, the platelets obtained in (3-5) above that were collected in the platelet product bag, and the culture of megakaryocytes collected in the waste fluid bag from which platelets had been removed (hereinafter, also collectively referred to as "raw materials" ), were used as the megakaryocytes and the culture thereof. Four samples (platelet-removed megakaryocyte cultures 1 to 4) that were separately prepared from the culture of megakaryocytes from which platelets had been removed were used.

Each of the raw materials were washed twice using a washing solution. An ACD-A solution was added to a Bicanate injection such that the concentration was about 20%(v/v), and the pH was adjusted to be within a range from 7.0 to 7.4 by adding NaOH. The resulting solution was used as the washing solution. After the washing, each of the raw materials was centrifuged at 2000xg at room temperature (about 25°C) for 10 minutes. After the centrifugation, the precipitate was collected, and was frozen using liquid nitrogen. Next, a cell lysis buffer was added to the frozen precipitate, and the precipitate was dissolved by shaking the mixture at 50 rpm at 4°C for 30 minutes. A solution obtained by adding a protease inhibitor cocktail (manufactured by RayBiotech, Cat.No.: AA-PI) to a commercially available cell lysis buffer (2× Cell Lysis Buffer manufactured by RayBiotech, Cat.No.: AA-LYS) was used as the cell lysis buffer.

The obtained lysate was centrifuged at 14000×g at 4°C for 5 minutes. After the centrifugation, the supernatant was collected as an example of a treated product. The total protein concentration in the treated product obtained from each of the raw materials was measured using Pierce^{™} (BCA Protein Assay Kit (manufactured by Thermo Fisher Scientific). As a result, in the case where the immortalized megakaryocyte strains were used, the amount of the total protein extracted from 2.2×10⁷ cells was 2.604 mg. In the case where the platelets were used, the amount of the total protein extracted from 2.5×10^{g} cells was 1.187 mg. Furthermore, in the case where the platelet-removed megakaryocyte cultures 1 to 4 were used, the amounts of the total proteins extracted from 1.97×10⁸ cells, 5.25×10⁸ cells, 3.64×10⁸ cells, and 6.22×10⁸ cells were 2.8, 5.944, 3.6, and 4.496 mg, respectively. After the total protein concentration was adjusted to 5 mg/ml, the concentrations (pg/total protein 5 mg) of growth factors (bFGF, IGFBP-1, IGFBP-2, PIGF, VEGF, GDF-15, AR, BMP-7, and HGF) and growth factor receptors (SCFR, EGFR, and VEGFR2) in each of the treated products were measured using Quantibody^{®} Human Growth Factor Array 1 (manufactured by RayBiotech). Table 2 below shows the results. Note that it can also be said that Table 2 below shows the concentrations per 5 mg of the total protein.

**Table 2**

| | Immortalized megakaryocytes | Platelets | Platelet-removed megakaryocyte culture 1 | Platelet-removed megakaryocyte culture 2 | Platelet-removed megakaryocyte culture 3 | Platelet-removed megakaryocyte culture 4 |
|---|---|---|---|---|---|---|
| AR | 1.22 | 3.68 | 5.92 | 54.30 | 15.63 | 49.93 |
| bFGF | 12095.43 | 8204.79 | 71688.60 | 64295.53 | 67623.46 | 58957.22 |
| BMP-7 | 7990.20 | 5963.72 | 4411.27 | 325.58 | 71.43 | 157.92 |
| GDF-15 | 7301.68 | 9028.55 | 13392.39 | 22623.63 | 19756.97 | 17166.80 |
| IGFBP-1 | 1209 | 4.87 | 31.32 | 38.13 | 194.83 | 18.03 |
| IGFBP-2 | 49374.40 | 435632.49 | 341979.83 | 168537.40 | 194034.44 | 26793.87 |
| PIGF | 26.58 | 6.60 | 71.01 | 44.90 | 23.52 | 66.87 |
| SCF | 2898.13 | 35.37 | 61.19 | 71.34 | 187.32 | 281.21 |
| VEGF | 228.55 | 472.63 | 1434.18 | 1218.30 | 1321.43 | 1299.05 |
| SCFR | 9440.46 | 953.60 | 2277.34 | 3784.83 | 4437.62 | 3593.33 |
| EGFR | 10.44 | 164.37 | 202.28 | 169.70 | 109.56 | 5.04 |
| VEGF R2 | 134.11 | 343.16 | 522.28 | 1185.16 | 1057.00 | 987.55 |

| | | | | | | |
|---|---|---|---|---|---|---|
| Unit: pg/mL | | | | | | |

### (5) Confirmation of Cell Proliferation-Promoting Activity

Human adipose tissue-derived mesenchymal stem cells were seeded on a 10-cm dish such that the number of cells was 2.4× 10⁵ to 4.8×10⁵ cells/10 ml culture medium/dish. Note that commercially available human adipose tissue-derived mesenchymal stem cells (manufactured by Takara Bio Inc., Cat. No.: C-12977) were subcultured twice, and were then collected and used as the mesenchymal stem cells. The culture medium was prepared by adding the freeze-thawed composition prepared from the platelet-removed megakaryocyte culture 2 to a Mesenchymal Stem Cell Growth Medium 2 (manufactured by Takara Bio Inc., Cat. No.: C-28009). The composition was added such that the concentration of the total protein derived from the composition in the culture medium was a predetermined concentration (0, 125, 250, or 500 µg/ml). Note that the culture medium was a maintenance culture medium.

After being seeded, the mesenchymal stem cells were cultured under a wet condition at 37°C and 5% CO₂ for 3 days. After the culture, the mesenchymal stem cells were collected, seeded under the same conditions, and cultured again under a wet condition at 37°C and 5% CO₂ for 3 days. Next, the cultured mesenchymal cells were collected, and the number of cells was counted. Then, the relative value of the number of collected cells was calculated using the number of seeded cells as a standard (1), and was taken as the relative value of proliferative activity. Also, time (doubling time) required for the cells to proliferate once was calculated based on the number of seeded cells and the number of collected cells. Table 2 shows the results.

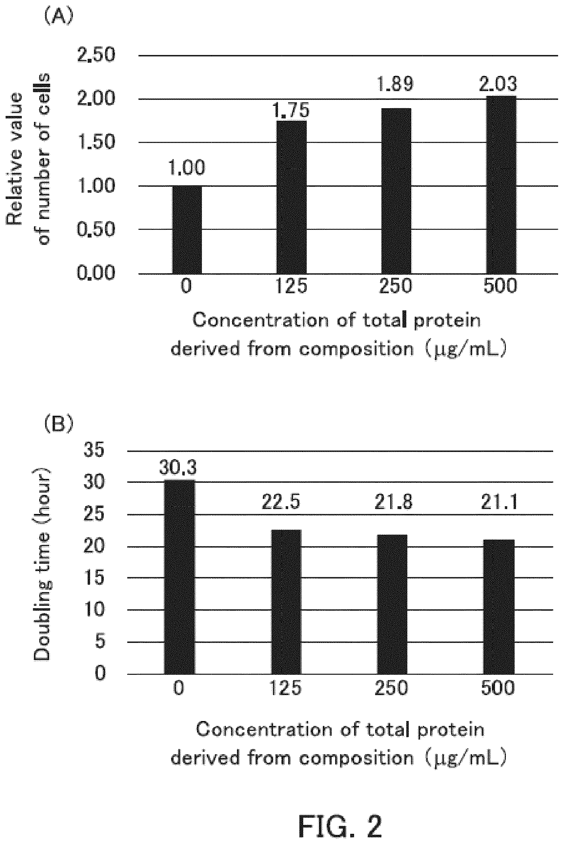

FIG. 2 shows graphs illustrating the cell-proliferative activity. FIG. 2(A) illustrates the proliferative activity, and FIG. 2(B) illustrates the doubling time. In FIG. 2(A), the horizontal axis indicates the concentration of the total protein derived from the composition, and the vertical axis indicates the relative value of the proliferative activity. In FIG. 2(B), the horizontal axis indicates the concentration of the total protein derived from the composition, and the vertical axis indicates the doubling time. The numerical values in the diagram each indicate the doubling time. As shown in FIG. 2(A), in the case where the composition of the present invention was added, the mesenchymal stem cell-proliferative activity increased depending on the concentration of the total protein derived from the composition. Although not shown in the diagram, after the third passage, the doubling time of mesenchymal stem cells to which the composition had not been added was 21 hours. As shown in FIG. 2(B), in the case where the composition of the present invention was not added, the doubling time of the mesenchymal stem cells was about 1.5 times longer. In contrast, as shown in FIG. 2(B), in the case where the composition of the present invention was added, time required for the mesenchymal stem cells to proliferate once was reduced depending on the concentration of the total protein derived from the composition. In the case where the mesenchymal stem cells were cultured in the culture medium to which the composition of the present invention had not been added, the doubling time was extended as the number of passages increased. In contrast, in the case where the composition of the present invention had been added, the extension of the doubling time was substantially inhibited. It was thus found that the composition of the present invention exhibited the cell proliferation-promoting activity and was capable of inhibiting a decrease in the proliferative activity.

### (6) Confirmation of Differentiation-Promoting Activity

The maintenance culture of the mesenchymal stem cells was carried out in the same manner as in (5) above. Next, the culture medium in each well was replaced with an equal amount of a differentiation culture medium (day 0 after the start of differentiation). The differentiation culture medium was prepared by adding the freeze-thawed composition prepared from the platelet-removed megakaryocyte culture 2 to a Mesenchymal Stem Cell Osteogenic Differentiation Medium (manufactured by Takara Bio Inc., Cat. No.: C-28013). The composition was added such that the concentration of the total protein derived from the composition in the differentiation culture medium was a predetermined concentration (0, 125, or 250 µg/ml), and the combination of the concentration of the total protein derived from the composition in the differentiation culture medium and the concentration of the total protein derived from the composition in the maintenance culture medium was as shown in Table 3 below.

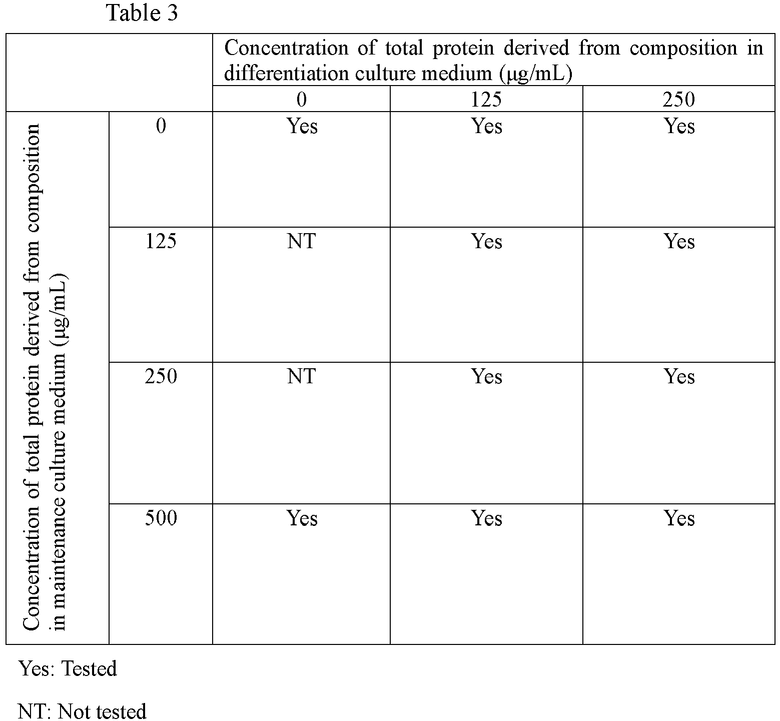

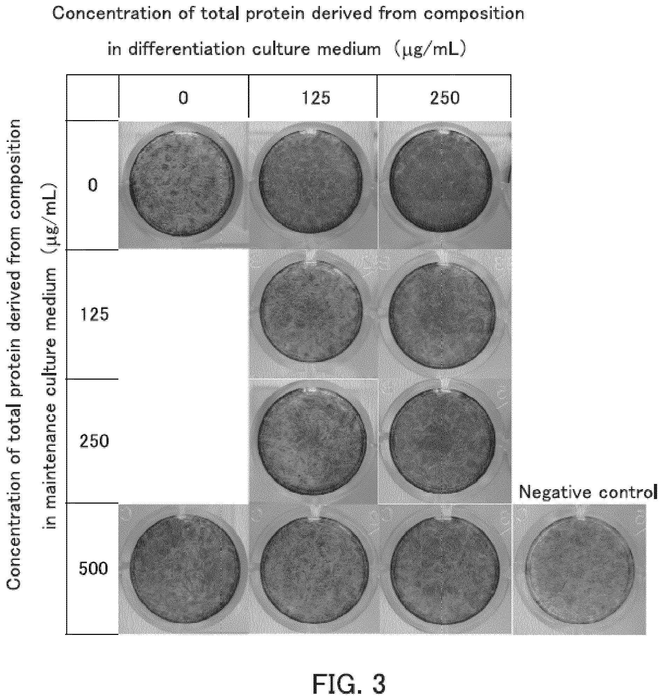

After being seeded, the mesenchymal stem cells were cultured under a wet condition at 37°C and 5% CO₂ for 14 days. During the culture, the culture medium was replaced every 3 or 4 days. On day 7 after the start of differentiation, the expression of alkaline phosphatase in each sample was confirmed through staining using a staining kit (TRACP & ALP double-stain Kit, manufactured by Takara Bio Inc., Cat. No.: MK300). A negative control was carried out in the same manner, except that only the maintenance culture medium was added instead of the differentiation culture medium. FIG. 3 shows the results.

FIG. 3 shows images obtained through ALP staining. As shown in FIG. 3, it was confirmed that all the samples were positive for ALP, and the differentiation to osteoblasts was induced in all the samples. Also, in the case where the composition of the present invention was added to the maintenance culture medium or the differentiation culture medium, ALP had intense staining in the cultured mesenchymal stem cells as compared with the case where the composition of the present invention was not added to the maintenance culture medium or the differentiation culture medium, and as compared with that of the negative control, thereby suggesting that the differentiation to osteoblasts was promoted.

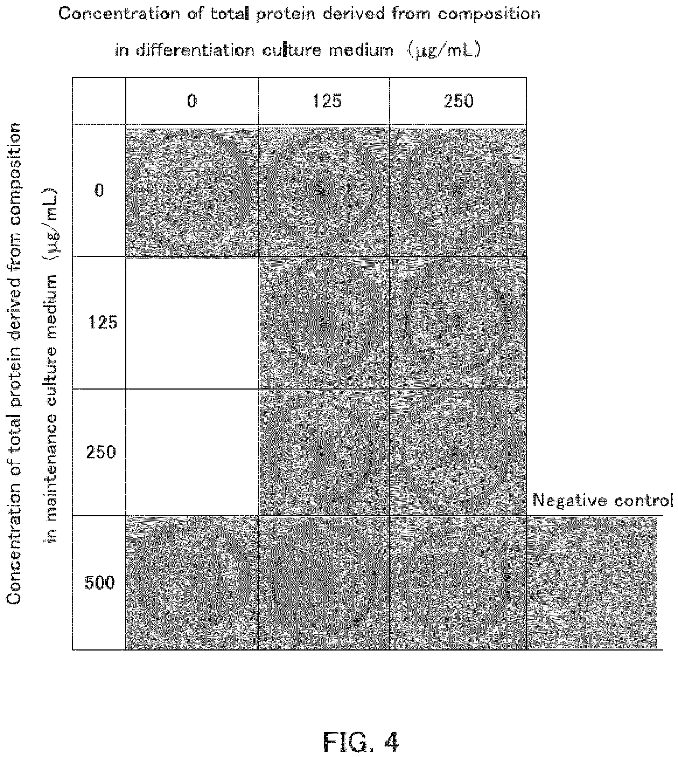

On day 14 after the start of differentiation, it was checked whether bone matrices were formed in each sample by staining calcium using alizarin red S. A negative control was carried out in the same manner, except that only the maintenance culture medium was added instead of the differentiation culture medium. FIG. 4 shows the results.

FIG. 4 shows images obtained through calcium staining. As shown in FIG. 4, substantially no calcium was stained in the sample in which the composition of the present invention was not added to both the maintenance culture medium and the differentiation culture medium, as well as in the negative control. In contrast, in the samples in which the composition of the present invention was added to at least one of the maintenance culture medium and the differentiation culture medium, calcium was stained, and it was thus confirmed that bone matrices were formed. That is to say, it was found that the composition of the present invention promoted the differentiation to osteoblasts.

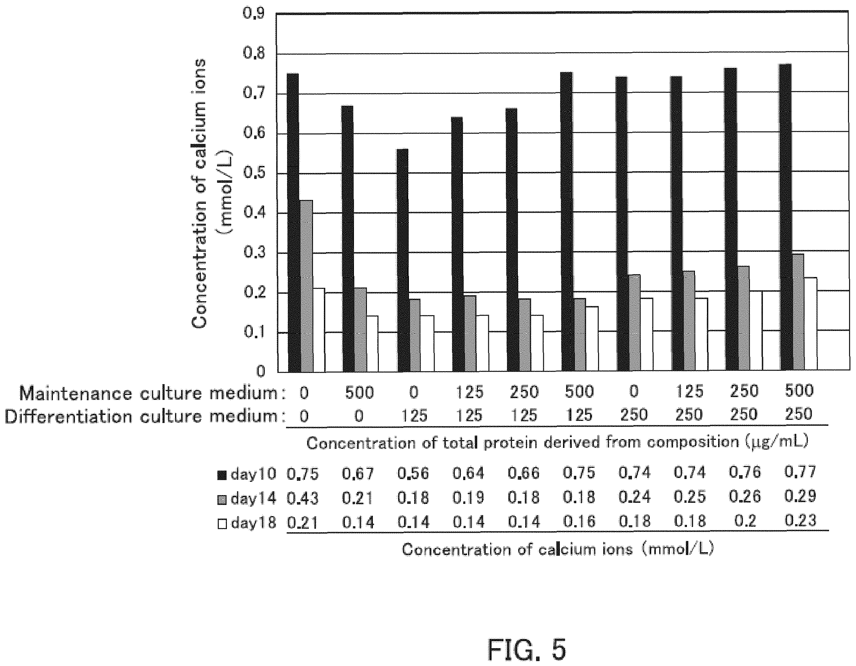

Furthermore, on days 10, 14, and 18 after the start of differentiation, the concentration of calcium ions in the culture medium was measured. The concentration of calcium ions in the culture medium was measured using an ion-selective electrode (BIOPROFILE^{®} FLEX, manufactured by NOVA BIOMEDICAL). Note that the concentration of calcium ions in the culture medium at the start of the culture was 0.88 mmol/l. In the case where the mesenchymal stem cells differentiate to osteoblasts and osteoblasts, bone matrices are formed using calcium in the culture medium. Accordingly, it is possible to evaluate the differentiation to osteoblasts and osteocytes using a decrease in the concentration of calcium ions as an indicator. FIG. 5 shows the results.

FIG. 5 is a graph illustrating the concentrations of calcium ions in the culture media. In FIG. 5, the horizontal axis indicates the concentrations of the total protein derived from the composition in the maintenance culture medium and the differentiation culture medium, and the actual measured values of the concentrations of calcium ions on the measurement days, and the vertical axis indicates the concentrations of calcium ions. As shown in FIG. 5, on day 10 after the start of differentiation, the concentration of calcium ions in the samples to which the composition of the present invention had been added were lower than or equal to that in the sample to which the composition of the present invention had not been added. On day 14 after the start of differentiation, the concentration of calcium ions in the samples to which the composition of the present invention had been added decreased noticeably compared with that in the sample to which the composition of the present invention had not been added. On day 18 after the start of differentiation, the concentration of calcium ions in the samples to which the composition of the present invention had been added were lower than or equal to that in the sample to which the composition of the present invention had not been added. It was found from these results that, when the composition of the present invention was added, differentiation to osteoblasts and osteocytes progressed early, and uptake of calcium from the culture medium was started earlier. It was found from these results that the composition of the present invention had activity to promote differentiation to osteoblasts.

### Example 2

It was checked whether the composition of the present invention had cell proliferation-promoting activity and activity to promote differentiation to osteoblasts.

### (1) Confirmation of Cell Proliferation-Promoting Activity

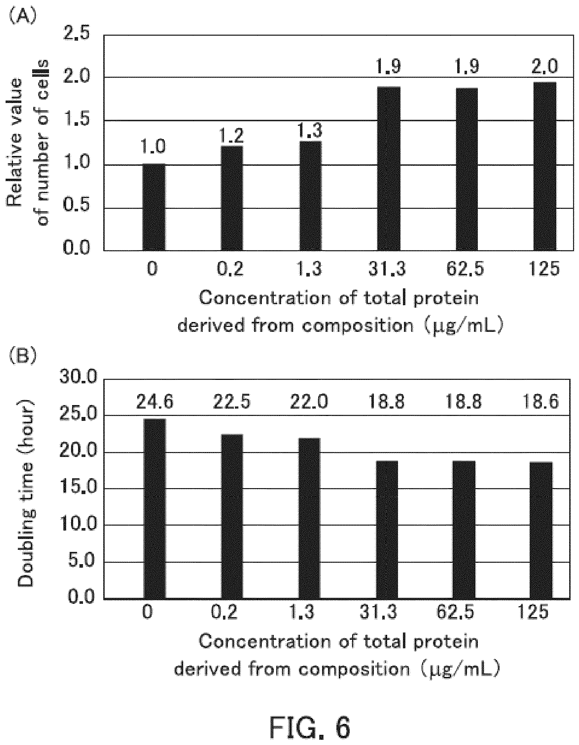

The mesenchymal stem cells were subcultured twice, and were then collected and cryopreserved. The cryopreserved cells were thawed and subcultured once, and were then used to calculate the proliferative activity and the doubling time in the same manner as in Example 1(5) above, except that the composition was added such that the concentration of the total protein derived from the composition in the maintenance culture medium was a predetermined concentration (0, 0.2, 1.3, 31.3, 62.5, or 125 µg/ml). FIG. 6 shows the results.

FIG. 6 shows graphs illustrating the cell-proliferative activity. FIG. 6(A) illustrates the proliferative activity, and FIG. 6(B) illustrates the doubling time. In FIG. 6(A), the horizontal axis indicates the concentration of the total protein derived from the composition, and the vertical axis indicates the relative value of the proliferative activity. In FIG. 6(B), the horizontal axis indicates the concentration of the total protein derived from the composition, and the vertical axis indicates the doubling time. As shown in FIG. 6(A), in the case where the composition of the present invention was added, the mesenchymal stem cell-proliferative activity increased depending on the concentration of the total protein derived from the composition. In particular, in the case where the concentration of the total protein was 31.3 µg/ml or more, the mesenchymal stem cell-proliferative activity increased noticeably. Although not shown in the diagram, after the third passage, the doubling time of the mesenchymal stem cells to which the composition had not been added was 17 hours. As shown in FIG. 6(B), in the case where the composition of the present invention was not added, the doubling time of the mesenchymal stem cells was about 1.5 times longer. In contrast, as shown in FIG. 6(B), in the case where the composition of the present invention was added, time required for the mesenchymal stem cells to proliferate once was reduced depending on the concentration of the total protein derived from the composition. In particular, in the case where the concentration of the total protein was 31.3 µg/ml or more, time required for the mesenchymal stem cells to proliferate once was reduced noticeably. In the case where the mesenchymal stem cells were cultured in the culture medium to which the composition of the present invention had not been added, the doubling time was extended as the number of passages increased. In contrast, in the case where the composition of the present invention had been added, the extension of the doubling time was substantially inhibited, and this effect was noticeable when the concentration of the total protein derived from the composition was set to 31.3 µg/ml or more. It was thus found that the composition of the present invention exhibited the cell proliferation-promoting activity and was capable of inhibiting a decrease in the proliferative activity.

### (2) Confirmation of Differentiation-Promoting Activity

The maintenance culture of the mesenchymal stem cells was carried out in the same manner as in Examples 2(1) above. Next, the culture medium in each well was replaced with an equal amount of a differentiation culture medium (day 0 after the start of differentiation). The differentiation culture medium was prepared by adding the freeze-thawed composition prepared from the platelet-removed megakaryocyte culture 2 to a mesenchymal stem cell osteogenic differentiation medium. The composition was added such that the concentration of the total protein derived from the composition in the differentiation culture medium was a predetermined concentration (0, 0.2, 1.3, 31.3, 62.5, or 125 µg/ml), and the combination of the concentration of the total protein derived from the composition in the differentiation culture medium and the concentration of the total protein derived from the composition in the maintenance culture medium was as shown in Table 4 below.

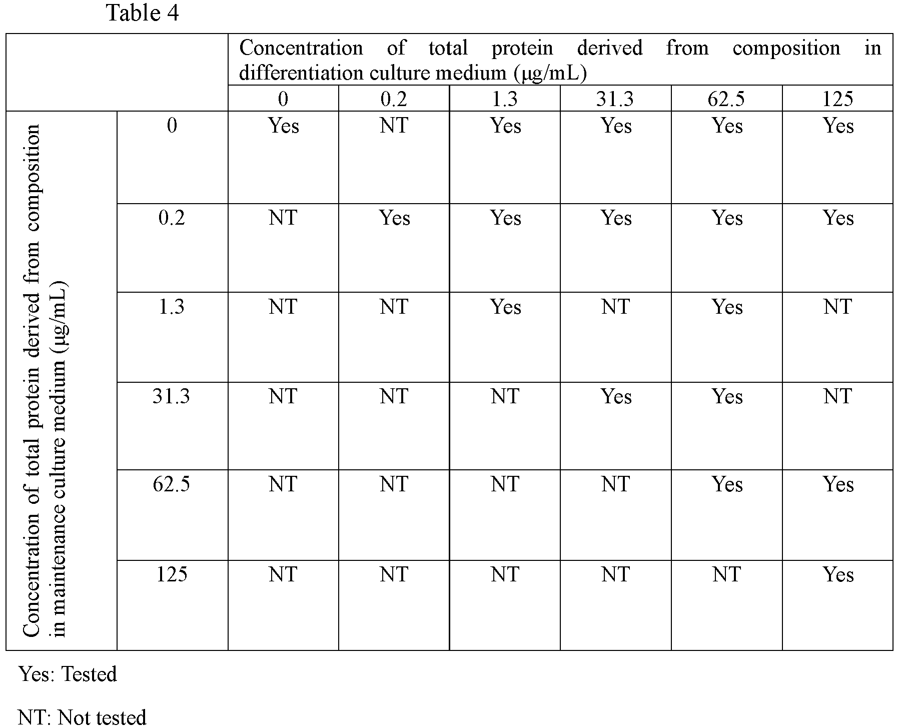

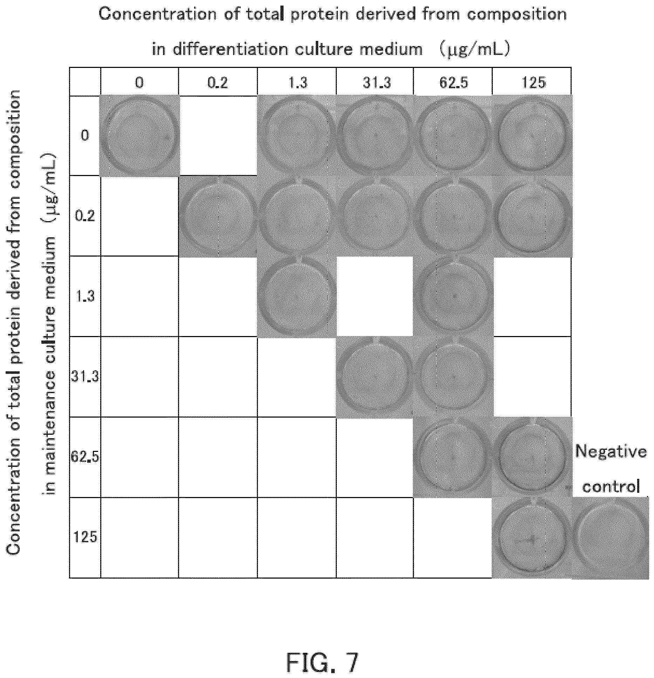

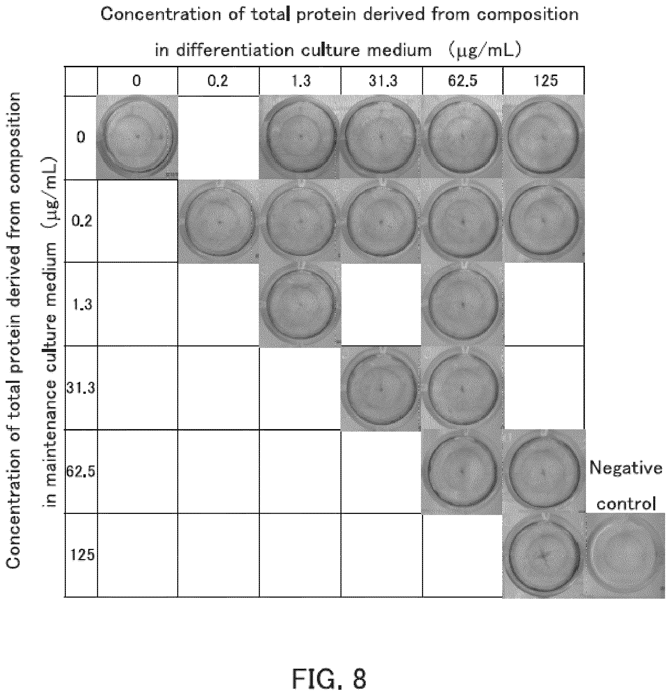

After being seeded, the cells were cultured in the same manner as in Examples 1(6) above. Next, on days 14 and 18 after the start of differentiation, it was checked whether bone matrices were formed in each sample by staining calcium in the same manner as in Example 1(6) above. FIGS. 7 and 8 show the results.

FIGS. 7 and 8 illustrate images obtained through calcium staining. As shown in FIGS. 7 and 8, substantially no calcium was stained in the sample in which the composition of the present invention was not added to both the maintenance culture medium and the differentiation culture medium, as well as in the negative control. In contrast, in the samples in which the composition of the present invention was added to at least one of the maintenance culture medium and the differentiation culture medium, calcium was stained, and it was thus confirmed that bone matrices were formed. That is to say, it was found that the composition of the present invention promoted the differentiation to osteoblasts.

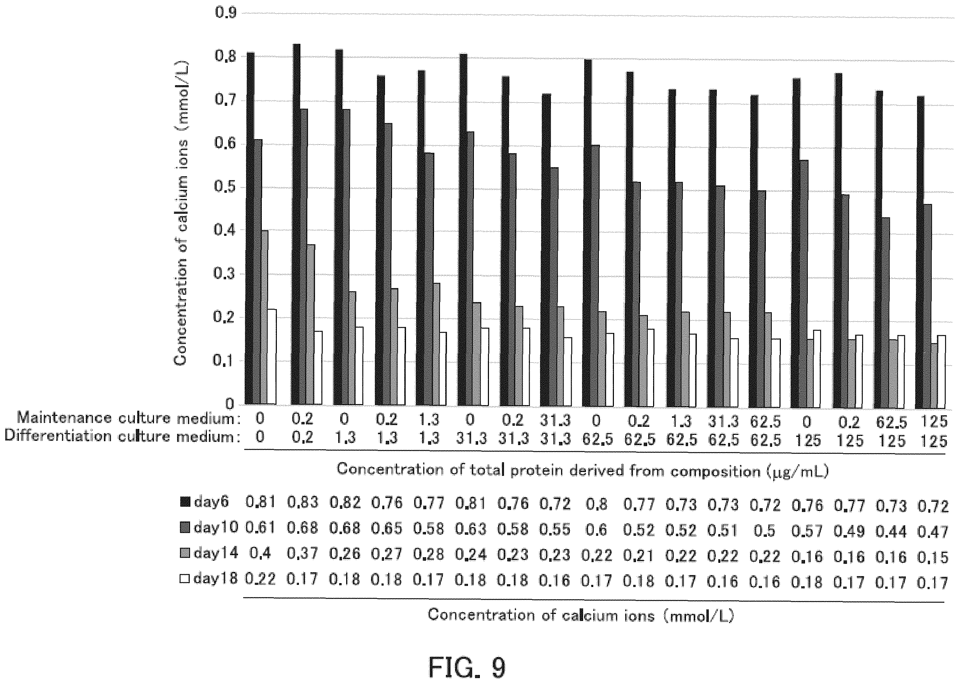

Furthermore, on days 6, 10, 14, and 18 after the start of differentiation, the concentration of calcium ions in the culture medium was measured in the same manner as in Example 1(6) above. FIG. 9 shows the results.

FIG. 9 is a graph illustrating the concentrations of calcium ions in the culture media. In FIG. 9, the horizontal axis indicates the concentrations of the total protein derived from the composition in the maintenance culture medium and the differentiation culture medium, and the actual measured values of the concentrations of calcium ions on the measurement days, and the vertical axis indicates the concentrations of calcium ions. As shown in FIG. 9, on days 6 and 10 after the start of differentiation, the concentration of calcium ions in the samples to which the composition of the present invention had been added were lower than or equal to that in the sample to which the composition of the present invention had not been added. On day 14 after the start of differentiation, the concentration of calcium ions in the samples to which the composition of the present invention had been added decreased compared with that in the sample to which the composition of the present invention had not been added. In particular, the concentration of calcium ions decreased noticeably in the sample in which the concentration of the total protein derived from the composition in the differentiation culture medium was 1.3 µg/ml. On day 18 after the start of differentiation, the concentration of calcium ions in the samples to which the composition of the present invention had been added were lower than or equal to that in the sample to which the composition of the present invention had not been added. It was found from these results that, when the composition of the present invention was added, differentiation to osteoblasts and osteocytes progressed early, and uptake of calcium from the culture medium was started earlier. It was also found that the composition of the present invention also exhibited the activity to promote differentiation to osteoblasts even when the concentration of the total protein derived from the composition was as low as 0.2 µg/ml. It was found from these results that the composition of the present invention had activity to promote differentiation to osteoblasts.

### Example 3

It was checked whether the composition of the present invention had cell proliferation-promoting activity and activity to promote differentiation to osteoblasts.

### (1) Confirmation of Cell Proliferation-Promoting Activity

Human bone marrow-derived mesenchymal stem cells (BMMSC, manufactured by PromoCell, Cat No. C-12974) were seeded on a 24-well plate such that the number of cells was 5×10⁴ cells/well, and then the cells were cultured for 7 days. The culture medium was replaced as follows: two-thirds of the culture medium was replaced with a new culture medium every 3 days. Note that in the instance where the culture is started from the above-mentioned seeding density, such a cell density reaches 80% confluency in about 7 days. A DMEM culture medium (low-glucose) containing 10% FBS and 1% anti-bacterial agent (Ab) was used as a basal culture medium for BMMSC.

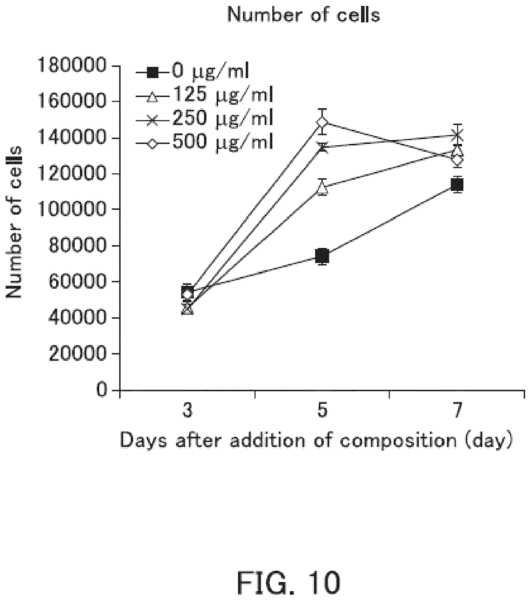

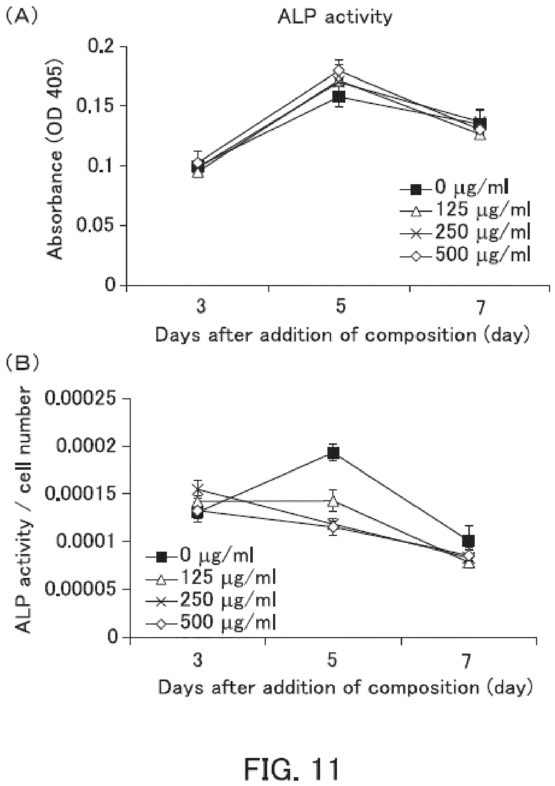

When 24 hours elapsed after the start of the culture, the composition derived from the platelet-removed megakaryocyte culture prepared in Examples 1(4) above was added such that the concentration of the total protein derived from the composition in the basal culture medium was a predetermined concentration (0, 125, 250, or 500 µg/ml). Then, on days 3, 5, or 7 after the addition of the composition, the number of cells was counted using a cell counting kit (WST8, Cell Counting Kit-8, manufactured by Dojindo, Cat No. 347-07621) in accordance with the attached protocol. Moreover, the ALP activity was examined by measuring the absorbance at 405 nm using p-nitrophenylphosphate (SIGMAFAST^{™} p-nitrophenylphosphate tablet, manufactured by SIGMA, Cat No. N1891). Then, the ALP activity value per cell was calculated. FIGS. 10 and 11 show the results.

FIG. 10 is a graph illustrating the number of cells. In FIG. 10, the horizontal axis indicates days after the addition of the composition, and the vertical axis indicates the number of cells. The concentrations in the diagram indicate the concentrations of total protein (the same applies hereinafter). As shown in FIG. 10, when the cells were cultured in the culture medium containing the composition of the present invention, the proliferation of the cells was promoted in a concentration-dependent manner. Note that the reason why the number of cells decreased on day 7 in the 500-µg/ml addition group is that the cells were in a confluent state on day 5.

Next, FIG. 11 shows graphs illustrating the ALP activity. In FIG. 11(A), the horizontal axis indicates days after the addition of the composition, and the vertical axis indicates the ALP activity (absorbance OD405). In FIG. 11(B), the horizontal axis indicates days after the addition of the composition, and the vertical axis indicates the ALP activity (ALP activity value per cell number). As shown in FIG. 11(B), the ALP activity per well reached a maximum on day 5, and decreased on day 7. Meanwhile, as shown in FIG. 11(A), the ALP activity per cell decreased over time under all the culture conditions. In general, MSCs differentiate as they proliferate, and therefore, the ALP activity thereof increases over time. However, in the presence of the composition of the present invention, the proliferation was strongly induced while an increase in the ALP activity was inhibited, and it was thus inferred that the induction of differentiation was inhibited. It was found from these results that the composition of the present invention exhibited cell proliferation-promoting activity.

### (2) Confirmation of Differentiation-Promoting Activity

Human bone marrow-derived mesenchymal stem cells were cultured for 7 days in the same manner as in Example 3(1) above.

After the culture, the culture medium in each well was replaced with the basal culture medium or a basal culture medium (differentiation culture medium) containing 100 µg/ml ascorbic acid and the composition as osteoblast differentiation factors, and the cells were cultured for 2 weeks to induce osteoblasts. The culture medium was replaced as follows: two-thirds of the culture medium was replaced with a new culture medium every 3 days. The composition was added such that the concentration of the total protein derived from the composition in the basal culture medium was a predetermined concentration (0, 125, 250, or 500 µg/ml). A composition derived from the platelet-removed megakaryocyte culture prepared in Example 1(4) above was used as the composition.

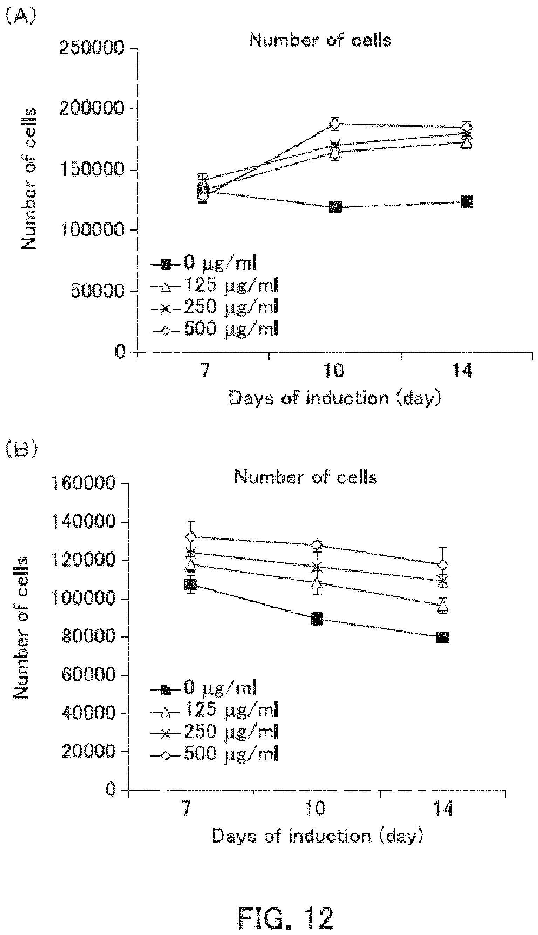

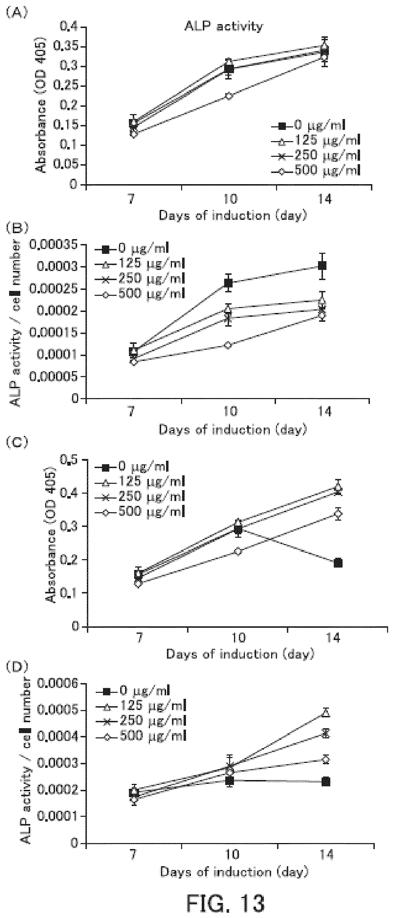

On days 7, 10, and 14 after the start of the induction, the number of cells and the ALP activity were measured. The number of cells was calculated using the cell counting kit in accordance with the attached protocol. The ALP activity was examined by measuring the absorbance at 405 nm using p-nitrophenylphosphate (SIGMAFAST^{™} p-nitrophenylphosphate tablet, manufactured by SIGMA, Cat No. N1891). Then, the ALP activity value per cell was calculated. FIGS. 12 and 13 show the results.

FIG. 12 is a graph illustrating the number of cells. In FIG. 12, the horizontal axis indicates days after the start of induction of osteoblast differentiation, and the vertical axis indicates the number of cells. As shown in FIG. 12(A), in the case where the osteoblast differentiation factors were not included, it was observed that the number of cells increased over time when the cells were cultured in the culture medium containing the composition of the present invention. On the other hand, as shown in FIG. 12(B), in the case where the osteoblast differentiation factors were included, a decrease in the number of cells over time was inhibited in a concentration-dependent manner when the cells were cultured in the culture medium containing the composition of the present invention.

Next, FIG. 13 shows graphs illustrating the ALP activity. In FIGS. 13(A) and 13(C), the horizontal axis indicates days after the start of induction of osteoblast differentiation, and the vertical axis indicates the ALP activity (absorbance OD405). In FIGS. 13(B) and 13(D), the horizontal axis indicates days after the addition of the composition, and the vertical axis indicates the ALP activity (ALP activity value per cell number). As shown in FIGS. 13(A) and 13(B), in the case where the osteoblast differentiation factors were not included, when the cells were cultured in the culture medium containing the composition of the present invention, the ALP activity increased over time, but a difference in the ALP activity from the culture medium of the comparative example that did not contain the composition of the present invention (0 µg/ml) was not observed. On the other hand, as shown in FIGS. 13(C) and 13(D), in the case where the osteoblast differentiation factors were included, when the cells were cultured in the culture medium containing the composition of the present invention, the ALP activity increased significantly on day 14 after the start of the induction of the differentiation compared with the cells cultured in the culture medium of the comparative example that did not contain the composition of the present invention (0 µg/ml). Note that it is inferred that an increase in the absorbance of the culture medium of the comparative example (0 µg/ml) on day 10 after the start of the induction of the differentiation is caused by an increase in the number of cells due to cell proliferation. It was found from these results that the composition of the present invention was also capable of promoting the induction of osteoblast differentiation in the system for inducing osteoblast differentiation using ascorbic acid. It was also found that the composition of the present invention promoted the induction of differentiation to osteoblasts induced by the osteoblast differentiation factors. Therefore, it can be said that the composition of the present invention had activity to promote differentiation to osteoblasts.

### Example 4

It was checked whether the composition of the present invention promoted osteogenesis and bone formation *in vivo.*

A model where osteogenesis is performed on the skull of a nude mouse was used as an osteogenic model. Specifically, a transplantation sample that included the composition of the present invention together with an artificial bone material was transplanted on the skull (beneath the periosteum) of a nude mouse (8-week-old BALB/cAJCL-nu/nu) under a general anesthesia (a mixture of three types of anesthetic agents). Specifically, the following four groups were set.

### - Negative control group (n=5)

A transplantation sample prepared using only 25 mg of β-TCP (β-tricalcium phosphate) granules (OSferion G1, manufactured by Olympus Terumo Biomaterials Corp.) was transplanted.

### - Positive control group (n=9)

25 mg of the β-TCP granules and platelet-rich plasma (PRP) were transplanted. A transplantation sample was transplanted that included the platelet-rich plasma prepared by concentrating the platelets in mouse peripheral blood such that the platelet concentration was 6 times as high as the platelet concentration in the peripheral blood.

### - Example 4A (n=5)

A transplantation sample was transplanted that was prepared using 25 mg of the β-TCP granules and a composition (about 2 mg of total protein) prepared from the platelet-removed megakaryocyte culture containing cells (1.65 × 10⁸ cells), the number of cells being as many as those in the positive control.

### - Example 4B (n=16)

A transplantation sample was transplanted that was prepared using 25 mg of the β-TCP granules and a composition prepared from the platelet-removed megakaryocyte culture containing cells (8.25×10⁸ cells), the number of cells being 5 times as many as those in the positive control.

The artificial bone materials were prepared as follows.

### - Negative control group

25 mg of sterile TCP was mixed with 50 µl of bovine fibrinogen (10 mg/ml) (Sigma, F8630) on Dappen glass, and the mixture was made into a gel by adding 5 µl of bovine thrombin (100 U/ml) (Sigma, T9549) thereto. Since the obtained gel had to be rapidly transplanted, these steps were carried out in an animal center.

### - Positive control group

In a low-protein-binding tube, 25 mg of the β-TCP granules were impregnated with 100 µl of the platelet-rich plasma (PRP). After the impregnation, the granules were mixed with 10 µl of autoserum (10 mg/ml), and the mixture was made into a gel by adding 5 µl of thrombin (100 U/ml) thereto. Since the obtained gel had to be rapidly transplanted, these steps were carried out in an animal center.

### - Example 4A

In a low-protein binding tube, 8 µl of the composition of the present invention and 32 µl of sterile water (DW) were mixed, and then 25 mg of TCP was impregnated with the mixture. After the impregnation, the resultant mixture was mixed with 10 µl of fibrinogen (10 mg/ml), and was then made into a gel by adding 5 µl of thrombin (100 U/ml) thereto. Since the obtained gel had to be rapidly transplanted, these steps were carried out in an animal center.

### - Example 4B

In a low-protein binding tube, 25 mg of TCP was impregnated with 40 µl of the composition of the present invention. After the impregnation, the resultant mixture was mixed with 10 µl of fibrinogen (10 mg/ml), and was then made into a gel by adding 5 µl of thrombin (100 U/ml) thereto. Since the obtained gel had to be rapidly transplanted, these steps were carried out in an animal center.

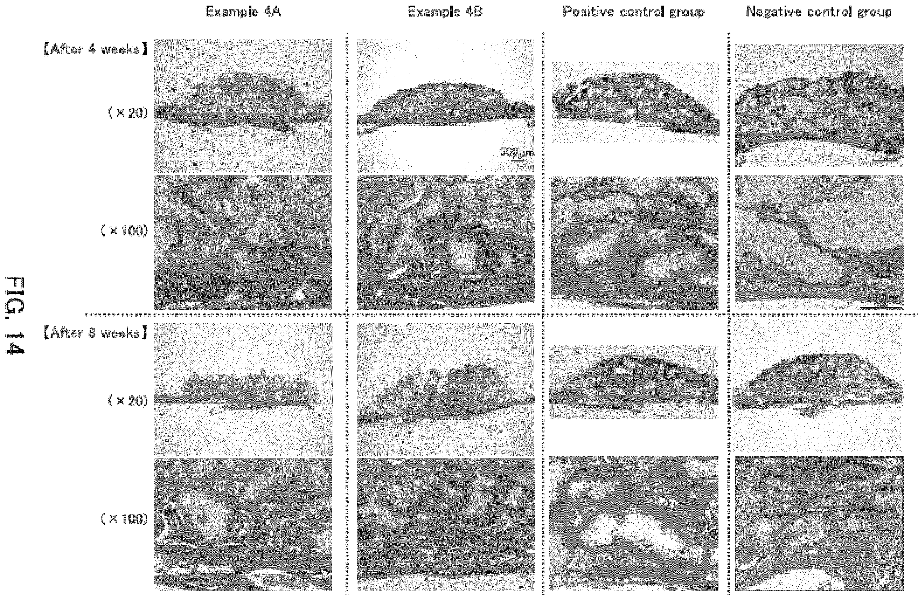

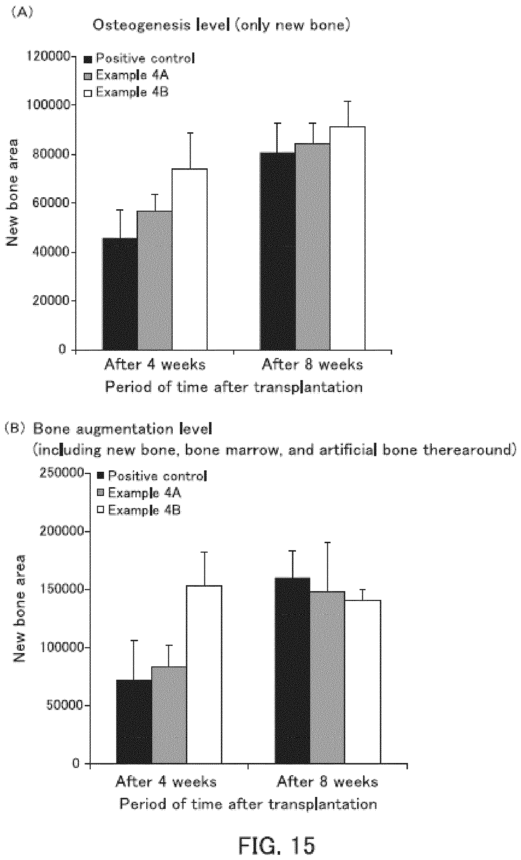

In the fourth or eighth week after the transplantation, the transplantation sample was excised together with a portion of the skull therearound, and was histologically observed through HE staining. In addition, the osteogenesis level and the bone formation level were examined by counting the pixels, using software (ImageJ), in the regions where osteogenesis or bone augmentation was observed. Note that the level of osteogenesis corresponded to the level of a newly formed bone in a region in the transplantation portion, and that the region subjected to the measurement for the bone formation level were a newly formed bone and a bone marrow, and an artificial bone therearound. Note that the osteogenesis level and the bone augmentation level were corrected using the osteogenesis level and the bone augmentation level of the negative control group. FIGS. 14 and 15 show the results.

FIG. 14 shows photographs illustrating osteogenesis. In FIG. 14, the photographs illustrate the results of Example 4A, Example 4B, the positive control group, and the negative control group in the order from left to right. In the photographs of each group, the upper photograph was taken using an objective lens with 20× magnification, and the lower photograph was taken using an objective lens with 100× magnification and is an enlarged photograph showing the region surrounded by a broken line in the upper photograph. As shown in FIGS. 14(A) to 14(C), in Examples 4(A) and 4(B) and the positive control, a bone was newly formed in a dark gray region around a light gray residual portion of an artificial bone.

Next, FIG. 15 shows graphs illustrating the osteogenesis level and the bone augmentation level. FIG. 15(A) illustrates the osteogenesis level, and FIG. 15(B) illustrates the bone augmentation level. In FIG. 15(A), the horizontal axis indicates a period of time after the transplantation of the transplantation material, and the vertical axis indicates the osteogenesis level. In FIG. 15(B), the horizontal axis indicates a period of time after the transplantation of the transplantation material, and the vertical axis indicates the bone augmentation level. As shown in FIGS. 14 and 15, in Example 4A, new bone formation and bone augmentation that included a bone marrow and an artificial bone around the new bone were observed to an extent equal to those in the positive control group, between the fourth week and the eighth week after the transplantation. Furthermore, in Example 4B, the osteogenesis level and the bone augmentation level were higher than those in the positive control group in the fourth week after the transplantation, but the osteogenesis level and the bone augmentation level were as high as those in the positive control group in the eighth week after the transplantation. It was inferred that the reason for this was that, in Example 4B, the osteogenesis level and the bone augmentation level had already reached plateaus of the osteogenesis levels and the bone augmentation levels comparable to the positive control group and Example 4A, in the fourth week after the transplantation.

### Example 5

It was checked whether the composition of the present invention had anti-inflammatory activity.

### (1) Activation of Immunocytes

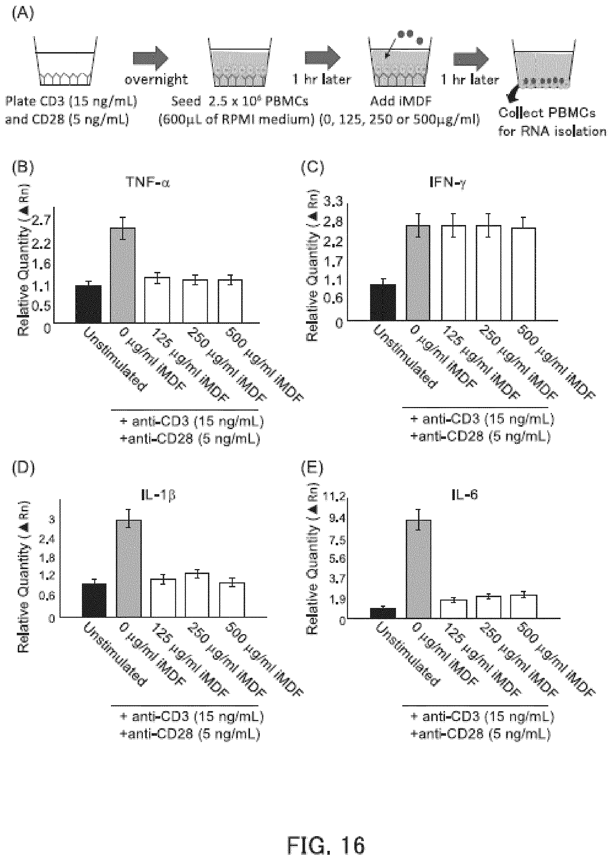

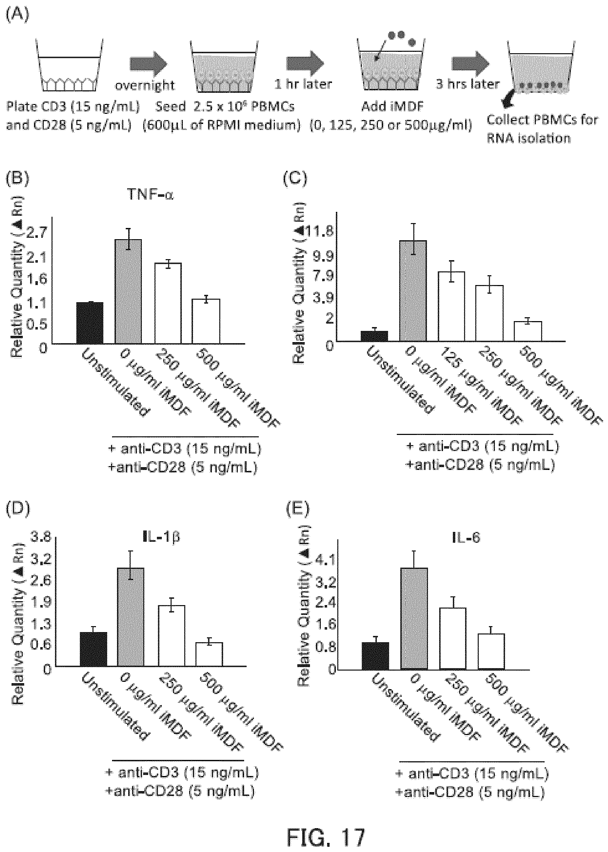

Immunocytes were activated as shown in FIGS. 16(A) and 17(A). Specifically, first, a phosphate buffer solution containing 15 ng/ml anti-human CD3 antibody (manufactured by eBioscience, Cat No.: 2045756) and 5 ng/ml anti-human CD28 antibody (manufactured by eBioscience, Cat No.: 1928813) was placed on a 24-well plate and was incubated overnight (about 12 hours), and thus these antibodies were immobilized on the plate. Next, peripheral blood monocytes were isolated, using Ficoll (Histopaque^{®} 1077, Sigma-Aldrich), from peripheral blood collected from a volunteer. The peripheral blood monocytes were seeded on the 24-well plate such that the number of monocytes was 2.5×10⁶ cells/well, and then the culture was started. An RPMI culture medium (manufactured by Gibco, Cat No.: 20621736) containing 10% FBS and 1% Ab was used as the culture medium for the peripheral blood monocytes.

A composition (iMDF) was added to each well 1 hour after the start of the culture such that the concentration of the total protein derived from the composition in the culture medium was a predetermined concentration (0, 125, 250, or 500 µg/ml). A composition derived from the platelet-removed megakaryocyte culture prepared in Example 1(4) above was used as the composition. After the addition of the composition, the cells were cultured for another 1 or 3 hours. After the culture, the peripheral blood monocytes were collected, and then total RNA was extracted from the peripheral blood monocytes using an mRNA extraction reagent (Trizol^{®}, manufactured by Thermo Fisher Scientific). cDNA was synthesized using the obtained RNA and a reverse transcriptase (SuperScript^{™} III First-Strand Synthesis System, manufactured by Thermo Fisher Scientific).

### (2) Measurement of Cytokine Expression Level

The obtained cDNA, the following primer sets, and a DNA polymerase (Takara-Taq, manufactured by Takara Bio Inc.) were used in a qPCR apparatus (Mx3000P QPCR System, manufactured by Agilent Technologies, Inc.) to measure the expression level of mRNA of each gene. After the measurement, the relative expression level of each gene was calculated based on the expression level of the internal standard gene (GAPDH gene). A negative control was carried out in the same manner, except that the anti-CD3 antibody and the anti-CD28 antibody were not immobilized (Unstimulated). FIGS. 16 and 17 show the results.

- Primer set for TNF-α
   Forward primer (Sequence ID No. 1)
   5' -AATGGCGTGGAGCTGAGA-3'
   Reverse primer (Sequence ID No. 2)
   5'-TAGACCTGCCCAGACTCGG-3'
- Primer set for IFN-α
   Forward primer (Sequence ID No. 3)
   5'-CTGTTACTGCCAGGACCCAT-3'
   Reverse primer (Sequence ID No. 4)
   5' -ACACTCTTTTGGATGCTCTGGT-3'
- Primer set for IL-1β
   Forward primer (Sequence ID No. 5)
   5'-CACAGACCTTCCAGGAGAAT-3'
   Reverse primer (Sequence ID No. 6)
   5' - TTCAACACGCAGGACAGGTA-3'
- Primer set for IL-6
   Forward primer (Sequence ID No. 7)
   5'-GTACATCCTCGACGGCATC-3'
   Reverse primer (Sequence ID No. 8)
   5' -AGCCACTGGTTCTGTGCCT-3'
- Primer set for GAPDH
   Forward primer (Sequence ID No. 9)
   5'-GGAGTCCACTGGCGTCTTCAC-3'
   Reverse primer (Sequence ID No. 10)
   5'-GCTGATGATCTTGAGGCTGTTGTC-3'

FIGS. 16 and 17 show schematic diagrams and graphs illustrating the overview of an experiment and cytokine gene-expression levels. In FIGS. 16(B) to 16(E) and FIGS. 17(B) to 17(E), the horizontal axis indicates the types of samples, and the vertical axis indicates the relative expression levels of the genes. As shown in FIGS. 17(B) to 17(E), the composition of the present invention inhibited the induction of expression of TNF-α, IFN-γ, IL-1β, and IL-6 caused by peripheral blood monocytes in a concentration-dependent manner 3 hours after the stimulation. On the other hand, as shown in FIGS. 16(B) to 16(E), the composition of the present invention inhibited the induction of expression of TNF-α, IL-1β, and IL-6 caused by peripheral blood monocytes in a concentration-dependent manner 1 hour after the stimulation. Note that the induction of expression of IFN-γ was not inhibited 1 hour after the stimulation while the induction of expression of IFN-γ was inhibited 3 hours after the stimulation, and it was thus found that the composition of the present invention exhibited the effect of inhibiting the induction of expression of IFN-γ at a later time as compared to that of the other inflammatory cytokines. The anti-CD3 antibody and the anti-CD28 antibody used in this example activate T cells, and it was thus inferred that the composition of the present invention inhibited activation of immunocytes such as T cells by inhibiting the induction of expression of cytokine genes.

### Example 6

It was checked whether the composition of the present invention promoted osteogenesis and bone formation at a defect site of a skull *in vivo.*

A model where osteogenesis is performed at a defect site of the skull of a nude rat was used as an osteogenic model. Specifically, a transplantation sample that included the composition of the present invention together with an artificial bone material was transplanted at 4-mm defect sites formed in the skull of a nude rat (8-week-old, F344/NJcl-rnu/nu) under a general anesthesia (a mixture of three types of anesthetic agents). Specifically, the following two groups were set.

### - Negative control group (n=17)

A transplantation sample prepared by impregnating an octacalcium phosphate (OCP) / collagen (Col) carrier having a diameter of 4 mm with PBS was transplanted.

### - Example 6 (n=8)

A transplantation sample was transplanted that was prepared by impregnating an OCP/Col carrier with, or causing an OCP/Col carrier to adsorb, a composition (about 25 mg of total protein) prepared using a platelet-removed megakaryocyte culture corresponding to platelet-rich plasma containing 8.25×10⁸ cells.

The artificial bone materials were prepared as follows.

### - Negative control group

OCP granules (having a diameter of 300 to 500 µm) and 3% porcine telocollagen were mixed, and then the mixture was freeze-dried. After the freeze-drying process, thermal crosslinking was performed to produce an OCP/Col carrier (OCP carrier, containing 77% OCP (weight ratio); diameter: 5 mm, thickness: 1 mm).

### - Example 6

An OCP carrier was impregnated with 20 µl of the composition of the present invention (corresponding to platelet-rich plasma containing 8.25×10⁸ cells), and thus an OCP carrier containing the composition of the present invention was obtained.

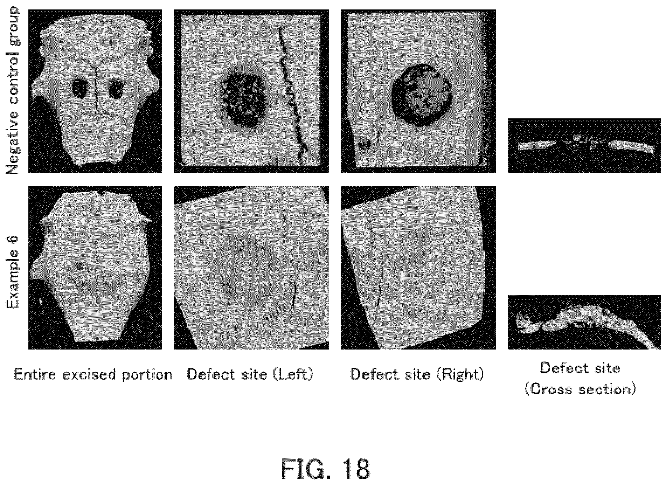

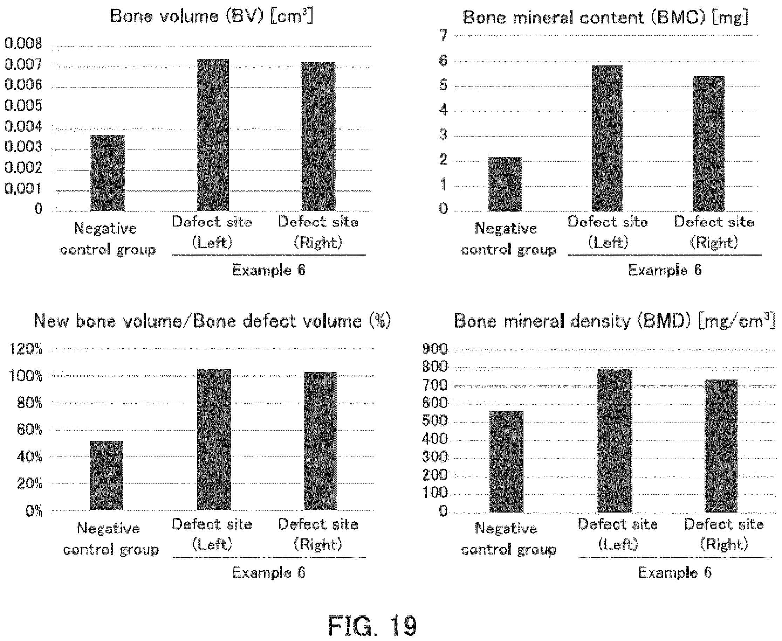

In the negative control group and Example 6, the transplanted sample was excised together with a portion of the skull therearound in the fourth or eighth week after the transplantation to the defect sites, and the obtained excised portions was subjected to µCT imaging. After the imaging, specimens were produced from the excised portion, and were histologically observed through HE staining or Masson trichrome staining and were subjected to immunostaining. The followings were evaluated: the bone volume and bone mineral content of a bone (new bone) that was newly formed from the transplanted material; the ratio (B_{R}/B_{S}) of the bone volume (B_{R}) of the bone that was newly formed from the transplanted material to the bone volume (Bs) of the portion of the bone excised when the defect site was formed; and the bone mineral density. In the evaluation above, 3D bone analysis software (TRI/3D-BON; manufactured by Ratoc System Engineering) was used to measure the bone form (bone volume: BV (cm³), bone mineral content: BMC (mg), bone mineral density: BMD (mg/cm³)). When the ratio (B_{R}/B_{S}) of the bone volume (B_{R}) of the bone that was newly formed from the transplanted material to the bone volume (Bs) of the portion of the bone excised when the defect site was formed was calculated, the bone defect volume was taken as 100%. FIGS. 18 and 19 show the results.

FIG. 18 shows photographs illustrating osteogenesis in the fourth week after the transplantation. In FIG. 18, the upper photographs illustrate the negative control group, and the lower photographs illustrate the results of the Example 6 group. The photographs in each group illustrate the entire excised portion, the left defect site, the right defect site, and a cross section of the excised portion that includes the defect site, in the order from left to right. It was found from the results shown in FIG. 18 that bones were newly formed at the defect sites of the skull in the Example 6 group.

FIG. 19 shows graphs illustrating the bone volumes, the bone mineral contents, the ratios between the bone defect volume and the new bone volume, and the bone mineral densities in the fourth week after the transplantation. FIG. 19(A) illustrates the bone volumes, FIG. 19(B) illustrates the bone mineral contents, FIG. 19(C) illustrates the ratios (B_{R}/B_{S}) between the bone defect volume and the new bone volume, and FIG. 19(D) illustrates the bone mineral densities. In FIG. 19(A), the horizontal axis indicates the type of experimental group, and the vertical axis indicates the bone volume BV (cm³). In FIG. 19(B), the horizontal axis indicates the type of experimental group, and the vertical axis indicates the bone mineral content (BMC) (mg). In FIG. 19(C), the horizontal axis indicates the type of experimental group, and the vertical axis indicates the ratio (%) between the bone defect volume and the new bone volume. In FIG. 19(D), the horizontal axis indicates the type of experimental group, and the vertical axis indicates the bone mineral density BMD (mg/cm³). As shown in FIG. 19, in Example 6, the bone volume, the bone mineral content, the ratio between the bone defect volume and the new bone volume, and the bone mineral density increased in both the left defect site and the right defect site as compared to those of the negative control group. It was found from the results above that the composition of the present invention was capable of forming a new bone in a defect site of the skull.

It was found from the descriptions above that the composition of the present invention was capable of inducing osteogenesis and bone augmentation, and osteogenesis and bone augmentation could be promoted by increasing the contents of the components of the composition.

As described above, the present invention has been described with reference to the embodiments and the examples, but the present invention is not limited to the above-described embodiments and examples. Various modifications that can be understood by a person skilled in the art can be made in the configurations and details of the present invention without departing from the scope of the present invention.

The present application claims the benefit of priority from Japanese Patent Application No. 2020-180042 filed on October 27, 2020, the entire disclosure of which is incorporated herein.

### Supplementary Notes

Some or all of the embodiments and examples given above can be described as in the following supplementary notes. However, the scope of the present invention is not limited thereto.

### Supplementary Note 1

An osteogenic composition comprising a treated product obtained by treating megakaryocytes or treating a culture thereof.

### Supplementary Note 2

The osteogenic composition according to supplementary note 1, wherein the treated product is an extract of megakaryocytes or an extract of a cellular fraction of a culture of megakaryocytes.

### Supplementary Note 3

The osteogenic composition according to supplementary note 1 or 2, wherein the treated product includes a basic fibroblast growth factor (bFGF) in an amount of 2000 to 20000 pg per 1 mg of a total protein.

### Supplementary Note 4

The osteogenic composition according to any one of supplementary notes 1 to 3, wherein the treated product includes an insulin-like growth factor-binding protein-2 (IGFBP-2) in an amount of 8000 to 80000 pg per 1 mg of a total protein.

### Supplementary Note 5

The osteogenic composition according to any one of supplementary notes 1 to 4, wherein the treated product includes a placental growth factor (PIGF) in an amount of 1 to 60 pg per 1 mg of a total protein.

### Supplementary Note 6

The osteogenic composition according to any one of supplementary notes 1 to 5, wherein the treated product includes a stem cell factor receptor (SCFR) in an amount of 200 to 2000 pg per 1 mg of a total protein.

### Supplementary Note 7

The osteogenic composition according to any one of supplementary notes 1 to 6, wherein the treated product includes a vascular endothelial growth factor (VEGF) in an amount of 20 to 800 pg per 1 mg of a total protein.

### Supplementary Note 8

The osteogenic composition according to any one of supplementary notes 1 to 7, wherein the treated product includes a vascular endothelial growth factor receptor 2 (VEGFR2) in an amount of 20 to 400 pg per 1 mg of a total protein.

### Supplementary Note 9

The osteogenic composition according to any one of supplementary notes 1 to 8, wherein the treated product includes a growth differentiation factor-15 (GDF-15) in an amount of 1000 to 10000 pg per 1 mg of a total protein.

### Supplementary Note 10

The osteogenic composition according to any one of supplementary notes 1 to 9, wherein the treated product includes a bone morphogenetic protein-7 (BMP-7) in an amount of 0 to 1000 pg per 1 mg of a total protein.

### Supplementary Note 11

The osteogenic composition according to any one of supplementary notes 1 to 10, wherein the treated product includes amphiregulin (AR) in an amount of 0 to 16 pg per 1 mg of a total protein.

### Supplementary Note 12

The osteogenic composition according to any one of supplementary notes 1 to 11, wherein the treated product includes an epidermal growth factor receptor (EGFR) in an amount of 0 to 60 pg per 1 mg of a total protein.

### Supplementary Note 13

The osteogenic composition according to any one of supplementary notes 1 to 12, wherein the treated product includes a hepatic growth factor (HGF) in an amount of 0 to 100 pg per 1 mg of a total protein.

### Supplementary Note 14

The osteogenic composition according to any one of supplementary notes 1 to 13, wherein the treated product includes an insulin-like growth factor-binding protein-1 (IGFBP-1) in an amount of 0 to 200 pg per 1 mg of a total protein.

### Supplementary Note 15

The osteogenic composition according to any one of supplementary notes 1 to 14, having cell proliferation-promoting activity.

### Supplementary Note 16

The osteogenic composition according to supplementary note 15, wherein the cell is a mesenchymal stem cell.

### Supplementary Note 17

The osteogenic composition according to any one of supplementary notes 1 to 16, having an ability to promote differentiation of an osteoblast progenitor cell to an osteoblast. Supplementary Note 18

The osteogenic composition according to supplementary note 17, wherein the osteoblast progenitor cell is a mesenchymal stem cell.

### Supplementary Note 19

The osteogenic composition according to any one of supplementary notes 1 to 18, wherein the culture of megakaryocytes is a culture from which platelets are removed.

### Supplementary Note 20

The osteogenic composition according to any one of supplementary notes 1 to 19, wherein the megakaryocytes are immortalized megakaryocytes.

### Supplementary Note 21

The osteogenic composition according to supplementary note 20, wherein the immortalized megakaryocytes are megakaryocytes that include a BMI1 gene, an MYC gene, and a Bcl-xL gene, which are exogenous genes.

### Supplementary Note 22

The osteogenic composition according to any one of supplementary notes 1 to 21, wherein the megakaryocytes are megakaryocytes induced *in vitro.*

### Supplementary Note 23

The osteogenic composition according to any one of supplementary notes 1 to 22, wherein the megakaryocytes are derived from pluripotent cells.

### Supplementary Note 24

The osteogenic composition according to supplementary note 23, wherein the pluripotent cells are induced pluripotent stem (iPS) cells.

### Supplementary Note 25

The osteogenic composition according to any one of supplementary notes 1 to 24,
wherein the treated product is obtained by
treating megakaryocytes or treating a culture thereof through
concentrating, drying, freezing, freeze-drying, solvent treatment, surfactant treatment, enzyme treatment, protein-fraction extraction treatment, ultrasonic treatment, and/or disruption treatment.

### Supplementary Note 26

The osteogenic composition according to supplementary note 25,
wherein the treated product is obtained by
removing platelets from the megakaryocytes or the culture thereof, and
treating the megakaryocytes or the culture thereof from which platelets have been removed.

### Supplementary Note 27

The osteogenic composition according to supplementary note 26,
wherein the treated product is obtained by
storing the megakaryocytes or the culture thereof from which platelets have been removed, and
treating the stored megakaryocytes or the stored culture thereof.

### Supplementary Note 28

The osteogenic composition according to supplementary note 27,
wherein the treated product is obtained by
storing the megakaryocytes or the culture thereof, and
disrupting the stored megakaryocytes or the stored culture thereof.

### Supplementary Note 29

An osteogenic kit comprising:
an osteogenic composition; and
a scaffold material for osteogenesis,
wherein the osteogenic composition is the osteogenic composition according to any one of supplementary notes 1 to 28.

### Supplementary Note 30

The kit according to supplementary note 29, wherein the scaffold material has biocompatibility and/or biodegradability.

### Supplementary Note 31

The kit according to supplementary note 29 or 30, wherein the scaffold material is constituted by at least one material selected from the group consisting of collagen, gelatin, albumin, keratin, dextran, carboxymethyl cellulose, xanthan gum, chitosan, chondroitin sulfate, heparin, hyaluronic acid, polyglycolic acid, polylactic acid, copolymers of polyglycolic acid and polylactic acid, polyhydroxybutyrate, polydioxanone, polycaprolactone, polybutylene succinate, calcium phosphate, hydroxyapatite, polyetherketone, and polyetheretherketone.

### Supplementary Note 32

The kit according to any one of supplementary notes 29 to 31, wherein the scaffold material has a porous structure.

### Supplementary Note 33

The kit according to any one of supplementary notes 29 to 32, comprising a physiologically active substance.

### Supplementary Note 34

The kit according to any one of supplementary notes 29 to 33, comprising at least one material selected from the group consisting of a vascular endothelial growth factor (VEGF), a platelet-derived growth factor (PDGF), an epidermal growth factor (EGF), a fibroblast growth factor (FGF), a hepatocyte growth factor (HGF), an insulin-like growth factor (IGF), a brain-derived neurotrophic factor (BDNF), a growth differentiation factor-5 (GDF5), an erythropoiesis-stimulating factor (EPO), a transforming growth factor (TGF), and a bone morphogenetic protein.

### Supplementary Note 35

An osteoblast differentiation-promoting composition comprising the osteogenic composition according to any one of supplementary notes 1 to 28.

### Supplementary Note 36

An immunocyte activation inhibitory composition comprising a treated product obtained by treating megakaryocytes or a culture thereof.

### Supplementary Note 37

The immunocyte activation inhibitory composition according to supplementary note 36, wherein the treated product is an extract of megakaryocytes or an extract of a cellular fraction of a culture of megakaryocytes.

### Supplementary Note 38

The immunocyte activation inhibitory composition according to supplementary note 36 or 37, wherein the treated product includes a basic fibroblast growth factor (bFGF) in an amount of 2000 to 20000 pg per 1 mg of a total protein.

### Supplementary Note 39

The immunocyte activation inhibitory composition according to any one of supplementary notes 36 to 38, wherein the treated product includes an insulin-like growth factor-binding protein-2 (IGFBP-2) in an amount of 8000 to 80000 pg per 1 mg of a total protein.

### Supplementary Note 40

The immunocyte activation inhibitory composition according to any one of supplementary notes 36 to 39, wherein the treated product includes a placental growth factor (PIGF) in an amount of 1 to 60 pg per 1 mg of a total protein.

### Supplementary Note 41

The immunocyte activation inhibitory composition according to any one of supplementary notes 36 to 40, wherein the treated product includes a stem cell factor receptor (SCFR) in an amount of 200 to 2000 pg per 1 mg of a total protein.

### Supplementary Note 42

The immunocyte activation inhibitory composition according to any one of supplementary notes 36 to 41, wherein the treated product includes a vascular endothelial growth factor (VEGF) in an amount of 20 to 800 pg per 1 mg of a total protein.

### Supplementary Note 43

The immunocyte activation inhibitory composition according to any one of supplementary notes 36 to 42, wherein the treated product includes a vascular endothelial growth factor receptor 2 (VEGFR2) in an amount of 20 to 400 pg per 1 mg of a total protein.

### Supplementary Note 44

The immunocyte activation inhibitory composition according to any one of supplementary notes 36 to 43, wherein the treated product includes a growth differentiation factor-15 (GDF-15) in an amount of 1000 to 10000 pg per 1 mg of a total protein.

### Supplementary Note 45

The immunocyte activation inhibitory composition according to any one of supplementary notes 36 to 44, wherein the treated product includes amphiregulin (AR) in an amount of 0 to 16 pg per 1 mg of a total protein.

### Supplementary Note 46

The immunocyte activation inhibitory composition according to any one of supplementary notes 36 to 45, wherein the treated product includes an epidermal growth factor receptor (EGFR) in an amount of 0 to 60 pg per 1 mg of a total protein.

### Supplementary Note 47

The immunocyte activation inhibitory composition according to any one of supplementary notes 36 to 46, wherein the treated product includes a hepatic growth factor (HGF) in an amount of 0 to 100 pg per 1 mg of a total protein.

### Supplementary Note 48

The immunocyte activation inhibitory composition according to any one of supplementary notes 36 to 47, wherein the treated product includes an insulin-like growth factor-binding protein-1 (IGFBP-1) in an amount of 0 to 200 pg per 1 mg of a total protein.

### Supplementary Note 49

The immunocyte activation inhibitory composition according to any one of supplementary notes 36 to 48, wherein the immunocyte is a T cell.

### Supplementary Note 50

The immunocyte activation inhibitory composition according to any one of supplementary notes 36 to 49, wherein the megakaryocytes are immortalized megakaryocytes.

### Supplementary Note 51

The immunocyte activation inhibitory composition according to any one of supplementary notes 36 to 50, wherein the megakaryocytes are megakaryocytes induced *in vitro.*

### Supplementary Note 52

An anti-inflammatory composition comprising the immunocyte activation inhibitory composition according to any one of supplementary notes 36 to 51.

### Supplementary Note 53

An osteoblast differentiation-promoting method comprising using the osteogenic composition according to any one of supplementary notes 1 to 28.

### Supplementary Note 54

The osteoblast differentiation-promoting method according to supplementary note 53, wherein the osteogenic composition is used *in vitro* or *in vivo.*

### Supplementary Note 55

An osteoblast differentiation-promoting method comprising using the osteoblast differentiation-promoting composition according to supplementary note 35.

### Supplementary Note 56

The osteoblast differentiation-promoting method according to supplementary note 55, wherein the osteoblast differentiation-promoting composition is used *in vitro* or *in vivo.*

### Supplementary Note 57

An immunocyte activation-inhibiting method comprising using the immunocyte activation inhibitory composition according to any one of supplementary notes 36 to 51.

### Supplementary Note 58

The osteoblast differentiation-promoting method according to supplementary note 57, wherein the immunocyte activation inhibitory composition is used *in vitro* or *in vivo.*

### Supplementary Note 59

An inflammation-inhibiting method comprising using the anti-inflammatory composition according to supplementary note 52.

### Supplementary Note 60

The inflammation-inhibiting method according to supplementary note 59, wherein the anti-inflammatory composition is used *in vitro* or *in vivo.*

### Supplementary Note 61

A composition for use to induce osteogenesis, including, as an active component, a treated product obtained by treating megakaryocytes or a culture thereof.

### Supplementary Note 62

A composition for use to promote differentiation of osteoblasts, including, as an active component, a treated product obtained by treating megakaryocytes or a culture thereof.

### Supplementary Note 63

A composition for use to inhibit immunocyte activation, including, as an active component, a treated product obtained by treating megakaryocytes or a culture thereof. Supplementary Note 64

A composition for use to inhibit inflammation, including, as an active component, a treated product obtained by treating megakaryocytes or a culture thereof.

### Industrial Applicability

As described above, with the present invention, it is possible to provide a composition having cell-derived osteogenic activity. Also, the composition of the present invention has, for example, cell proliferation-promoting activity and activity to promote differentiation to osteoblasts. Accordingly, the present invention is very useful in the fields of medicine, regenerative medicine, and the like.

## Claims

1. An osteogenic composition comprising:
a treated product obtained by treating megakaryocytes or treating a culture thereof.

2. The osteogenic composition according to claim 1,
wherein the treated product is an extract of megakaryocytes or an extract of a cellular fraction of a culture of megakaryocytes.

3. The osteogenic composition according to claim 1 or 2,
wherein the treated product comprises a basic fibroblast growth factor (bFGF) in an amount of 2000 to 20000 pg per 1 mg of a total protein.

4. The osteogenic composition according to any one of claims 1 to 3,
wherein the treated product comprises an insulin-like growth factor-binding protein-2 (IGFBP-2) in an amount of 8000 to 80000 pg per 1 mg of a total protein.

5. The osteogenic composition according to any one of claims 1 to 4,
wherein the treated product comprises a placental growth factor (PIGF) in an amount of 1 to 60 pg per 1 mg of a total protein.

6. The osteogenic composition according to any one of claims 1 to 5,
wherein the treated product comprises a stem cell factor receptor (SCFR) in an amount of 200 to 2000 pg per 1 mg of a total protein.

7. The osteogenic composition according to any one of claims 1 to 6,
wherein the treated productcomprises a vascular endothelial growth factor (VEGF) in an amount of 20 to 800 pg per 1 mg of a total protein.

8. The osteogenic composition according to any one of claims 1 to 7,
wherein the treated product comprises a vascular endothelial growth factor receptor 2 (VEGFR2) in an amount of 20 to 400 pg per 1 mg of a total protein.

9. The osteogenic composition according to any one of claims 1 to 8,
wherein the treated product comprises a growth differentiation factor-15 (GDF-15) in an amount of 1000 to 10000 pg per 1 mg of a total protein.

10. The osteogenic composition according to any one of claims 1 to 9,
wherein the treated product comprises amphiregulin (AR) in an amount of 0 to 16 pg per 1 mg of a total protein.

11. The osteogenic composition according to any one of claims 1 to 10,
wherein the treated product comprises an epidermal growth factor receptor (EGFR) in an amount of 0 to 60 pg per 1 mg of a total protein.

12. The osteogenic composition according to any one of claims 1 to 11,
wherein the treated product comprises a hepatic growth factor (HGF) in an amount of 0 to 100 pg per 1 mg of a total protein.

13. The osteogenic composition according to any one of claims 1 to 12,
wherein the treated product comprises an insulin-like growth factor-binding protein-1 (IGFBP-1) in an amount of 0 to 200 pg per 1 mg of a total protein.

14. The osteogenic composition according to any one of claims 1 to 13, having cell proliferation-promoting activity.

15. The osteogenic composition according to claim 14,
wherein the cell is a mesenchymal stem cell.

16. The osteogenic composition according to any one of claims 1 to 15, wherein the osteogenic composition has an ability to promote differentiation of an osteoblast progenitor cell to an osteoblast.

17. The osteogenic composition according to claim 16,
wherein the osteoblast progenitor cell is a mesenchymal stem cell.

18. The osteogenic composition according to any one of claims 1 to 17,
wherein the megakaryocytes are immortalized megakaryocytes.

19. The osteogenic composition according to any one of claims 1 to 18,
wherein the megakaryocytes are megakaryocytes induced *in vitro.*

20. An osteogenic kit comprising:
an osteogenic composition; and
a scaffold material for osteogenesis,
wherein the osteogenic composition is the osteogenic composition according to any one of claims 1 to 19.
